# EUROPEAN PATENT SPECIFICATION

(11) **EP 1 738 765 B1**
(45) Date of publication and mention of the grant of the patent: **05.10.2011**
(21) Application number: 05721586.5
(22) Date of filing: 22.03.2005
(51) Int. Cl.: A61K 39/39

(54) **ANTIGEN-DRUG VEHICLE ENABLING TRANSMUCOSAL AND TRANSDERMAL ADMINISTRATION AND METHOD OF INDUCING MUCOSAL IMMUNITY, MUCOSAL VACCINE AND DDS USING THE SAME**
ANTIGEN-ARZNEIVEHIKEL FÜR DIE TRANSMUKOSALE UND TRANSDERMALE VERABREICHUNG UND VERFAHREN ZUR AUSLÖSUNG VON SCHLEIMHAUTIMMUNITÄT, SCHLEIMHAUT-VAKZINE UND DDS UNTER VERWENDUNG DESSELBEN
VEHICULE DE MEDICAMENT ANTIGENIQUE PERMETTANT L'ADMINISTRATION TRANSMUQUEUSE ET TRANSDERMIQUE ET PROCÉDÉ D'INDUCTION D'IMMUNITÉ MUQUEUSE, VACCINS PAR VOIE MUQUEUSE ET DDS

(30) Priority: 05.04.2004 JP 2004111249; 21.04.2004 JP 2004125036
(43) Date of publication of application: 03.01.2007
(73) Proprietor: The University of Tokushima, Tokushima-shi, Tokushima 770-0855 (JP)
(72) Inventor: Kido, Hiroshi, University of Tokushima, Tokushima-shi, Tokushima 770-0042 (JP); Mizuno, Dai, University of Tokushima, Tokushima-shi, Tokushima 770-0042 (JP)
(74) Representative: Owen, Deborah Jane
(86) International application number: PCT/JP2005/005659
(87) International publication number: WO 2005/097182

(56) References cited:
- EP-A- 0 976 403
- JP-A- 6 009 428
- US-A1- 2002 155 151
- VAN IWAARDEN J.F. ET AL: 'Alveolar Macrophages, Surfactant Lipids, and Surfactant Protein B Regulate the Induction of Immune Responses via the Airways' AM. J. RESPIR. CELL MOL. BIOL. vol. 24, 2001, pages 452 - 458, XP002989570
- AUGUSTO L.A. ET AL: 'Cellular Antiendotoxin Activities of Lung Surfactant Protein C in Lipid Vesicles' AMERICAN JOURNAL OF RESPIRATORY AND CRITICAL CARE MEDICINE vol. 168, 2003, pages 335 - 341, XP002989571
- AUGUSTO L.A. ET AL: 'Interaction of Pulmonary Surfactant Protein C with CD14 and Lipopolysaccharide' INFECTION AND IMMUNITY vol. 71, no. 1, January 2003, pages 61 - 67, XP002989572
- SCHRAM V. ET AL: 'Non-cooperative effects of lung surfactant proteins on early adsorption to an air/water interface' BIOCHIMICA ET BIOPHYSICA ACTA vol. 1616, 2003, pages 165 - 173, XP004464814

## Description

### Technical Field

The present invention relates to an antigen-drug vehicle enabling transmucosal and transdermal administration, and more specifically, to a method of inducing mucosal immunity which causes preferential and effective, particularly selective production of an antigen-specific secretory immunoglobulin A, a mucosal vaccine, prevention or treatment of allergy which is characterised by using the vehicle for desired antigen. Also disclosed herein is a drug delivery system which is characterized by using the vehicle for desired drug.

### Background Art

Conventional inactivated vaccine, toxoid, and the like are known to have the following drawbacks:

### (1) Lack of defense against infection in natural infection route

The natural infection route of bacterial, virus, and the like is, for example, the mucosal membrane of the nasal cavity, the trachea, the intestinal tract, and the like, whereas the vaccine inoculation route is subcutaneous, intramuscular, and the like, which is different from the route described above. It is desired to achieve defense against infection by an inoculation route fit for the actual conditions of the natural infection, particularly defense against infection at the mucosal membrane from vaccine administration via the mucosal membrane.

### (2) Low mucosal immunity

In the subject for vaccine inoculation, immunoglobulin G (hereinafter, simply referred to as "IgG" or "IgG antibody") is mainly produced into the blood, and humoral immunity is induced. However, immunoglobulin A (hereinafter, simply referred to as "IgA" or "IgA antibody") which takes charge of mucosal immunity, is nearly not produced, and therefore, establishment of mucosal immunity is not expected. Furthermore, necessity and effectiveness of IgA antibody are as follows: IgA antibody plays a very important roll in clinical immunity by taking charge of mucosal immunity, i.e., defense against infection at the mucosal membrane which is the entrance to infection onto the respiratory organs such as the nasal cavity and the trachea by spray or air, and infection onto the intestinal tract by oral. Furthermore, IgA antibody has cross immunity, i.e., cross-neutralization activity, and broad spectrum for infection defense as much, and also has defense against infection for variant antigen, whereas IgG antibody has high specificity for antigen, but has narrow spectrum for infection defense, leading to nearly not being effective for infection defense against variant antigen.

### (3) Necessity of additional vaccination and added expense

Since IgG antibody is produced low only by one injection of the first immunization, and certain effects cannot be expected, it is required to increase blood IgG antibody value by additional inoculation, so-called booster inoculation of once or more times, based on the conditions of IgG antibody retention later. Therefore, repeated expense and effort are required, and on top of that, the case is often found where the chance of the booster inoculation is not effective for young children, especially infants two-years old or less, who is likely to depart from the chance of the booster inoculation, though the effects are recognized for the elderly, adults, and school children, who are benefited from the chance.

In other words, conventional inactivated vaccine, toxoid, and the like induces mainly production of blood IgG antibody in the subject for vaccine inoculation, and also brings actions and effects of increasing humoral immunity, being recognized for its efficacy. However, it has low performance of inducing IgA antibody production or mucosal immunity, showing the limit about sufficient function and effects for defense against natural infection. From such circumstances, there have been many trials from various sides so far to resolve the drawbacks of the conventional vaccines. For example, it includes improvement of the vaccine antigen in quality or quantity, experimental production of live vaccine replacing inactivated vaccine, development of new inoculation route, mucosal vaccine, and the like, screening of adjuvant, which elevates humoral immunity and cause maintenance thereof, development of mucosal immunity adjuvant, and the like. However, development of mucosal vaccine which is safe and effective has not been achieved.

Hereinafter, development of mucosal vaccine will be explained.

### (1) Increase of the amount of the vaccine antigen

A trial has been conducted of increasing the amount of the vaccine antigen which is inoculated subcutaneously or intramuscularly, or increasing the amount of IgG and IgA antibodies, which are secreted to the mucosal membrane. For example, a method wherein neuraminidase of the virus membrane protein is added to and mixed with conventional inactivated influenza vaccine to increase antibody production amount, or a method wherein MF59 is added and mixed as an adjuvant, and the like has been tried. However, these methods have been found to have disadvantages such as incurrence of pain, strong adverse reaction, and the like.

### (2) Vaccine of nasal administration type

A method has been tried wherein liquid split antigen is directly inoculated nasally for infection defense by IgA antibody, which is considered most effective, but the fact is pointed out that IgA production amount is small. In order to elevate IgA antibody production ability, there has been a trial that Escherichia coli heat-labile live toxin or cholera toxin is added to and mixed as an adjuvant with the split antigen to elevate mucosal immunity response, i.e., IgA antibody production ability. However, from the circumstances that the safety of the toxin as an adjuvant has been not proved, the trial treatment has been stopped, and not been put to practical use.

### (3) Live vaccine using cold-attenuated strain which can be inoculated into the nasal cavity

A method is put to practical use wherein cold-attenuated influenza virus strain, which has optimal growth temperature of 25°C, and does not grow at 39°C, but the risk of toxicity recovery cannot be denied as the mechanism of attenuation of the parent cold-attenuated strain is not clear. In addition, since the active ingredient of the vaccine is a live virus, it has high invasion force into cells and excellent in initialization of immunity, but mild influenza symptoms often incur, so defects have been found that it cannot be used for human who has high risk of increase in severity when infected by influenza, elderly people, and the like.

### (4) Other vaccines

Developments of a vector vaccine which has vaccinia virus as a virus vector, attenuated live vaccine by reverse genetics, DNA vaccine which uses DNA or cDNA as an active ingredient, have been in progress in lab level, but not put to practical use.

Furthermore, development of immunity adjuvant will be explained below.

### (1) Immunity adjuvant

The immunity adjuvant is a general name of a substance which has regulation activity such as reinforcement or inhibition of immune response, and largely divided into two kinds: a substance which is related to a dosage form for the purpose of sustained-release, retention, and the like of antigen within the subject for inoculation, and a substance which helps elevation inhibition, and the like of immune response. Between them, as the former, i.e., as the adjuvant for dosage form, for example, vaccine or toxoid with use of aluminum phosphate, alum, and the like has been already put to practical use. However, the latter, i.e., the adjuvant which helps reinforcement/elevation of immune response has not been known yet to be put to practical use. For example, BCG live bacteria derived from bacteria, BCG-CWS, endotoxin, glucan, and the like, synthesized MDP, levamisole, Poly I-Poly C, bestatin, and the like, and interferons such as cytokines, TNF, CSF, and the like have been known, but it is considered that guarantee for the safety and efficacy is needed for the practical use of them, by the reasons of insufficient effects, and the like for adjuvant diseases such as arthritis, chronic arthritic rheumatism, hyper-γ-globulinemia, anemia, and the like. In addition, a technique is known (Patent Document 1) using a pulmonary surfactant/protein derived from a higher animal as an adjuvant in order to enhance induction of humoral immunity, but it has not known to be put to practical use.

### (2) Development of adjuvant for mucosal immunity

Various adjuvants have been developed, for example, pertussis toxin B oligomer (Patent Document 2), cholera toxin (Patent Document 3), Escherichia coli heat-labile enterotoxin B subunit (LTB) (Patent Document 4), starch particles (Patent Document 5), cholera toxin B chain protein (CTB) (Patent Document 6), B subunit of verotoxin 1 (Patent Document 7), oligonucleotide (Patent Document 8), interleukin 12 (Non-Patent Document 1), and the like. However, they have not been put yet to practical use.

As described above, necessity has been recognized widely and deeply for replacement of the conventional vaccine which is inoculated subcutaneously, intramuscularly, and the like with a mucosal vaccine which induces production of IgA antibody in the mucosal membrane, which is a natural infection route of virus. Particularly, as a vaccine of next generation in the 21 century, so-called mucosal vaccine, which induces production of IgA antibody, and local immunity or mucosal immunity, is expected and hoped worldwide to be developed and put to practical use, but not yet achieved. The reason is considered to be in the fact that safe and effective adjuvant, which imparts the function of inducing production of IgA antibody, and local immunity or mucosal immunity to vaccine, has not specified or established.
Patent Document 1: JP-T-2002-521460
Patent Document 2: JP-A-3-135923
Patent Document 3: JP-T-10-500102
Patent Document 4: JP-T-2001-523729
Patent Document 5: JP-T-2002-50452
Patent Document 6: JP-A-2003-116385
Patent Document 7: JP-A-2003-50452
Patent Document 8: The pamphlet of PCT WO 00/20039
Patent Document 9: EP-A-0 976 403 discloses the use of a pulmonary surfactant or a component thereof as an adjuvant.
Patent Document 10: US 2002/155151 A1 discloses transdermal, oral and intraveneous formulations of 2,3-dimethoxy-5-methyl-6-decaprenyl-1,4-benzoquinone (coenzyme Q10), which contain an effective amount of pulmonary surfactant.
Non-Patent Document 1: pp. 4780-4788, vol. 71, 2003, Infection and Immunity
Non-Patent Document 2: pp. 2-11, vol. 10, 2004, Journal of neonatal Nursing
Non-Patent Document 3: pp. 9-14, vol. 74 (suppl. 1), 1998, Biology of the Neonate
Non-Patent Document 4: pp. 452-458, vol. 24, 2001, American Journal of Respiratory Cell and Molecular Biology

### Disclosure of the Invention

The present invention has the following objects: specifically, to impart the function of inducing production of IgA antibody, and local immunity or mucosal immunity: development of safe and effective technique therefor; conversion from conventional humoral immunity vaccine to safe and effective mucosal immunity vaccine: and prevention and treatment of allergy.

As means to solve the objects, the present invention provides a mucosal vaccine comprising: (i) an antigen-drug vehicle, which is a complex comprising: a pulmonary surfactant protein B or a fragment of protein B that is a peptide comprising the amino acid sequence of SEQ ID No. 2. 7, 8, 10, 11, 12, 13, 14, 15, 16, 17, 18, 19 or 20, a pulmonary surfactant protein C or a fragment of protein C that is a peptide comprising the amino acid sequence of SEQ ID No. 4, 9, 21 or 6, and at least one lipid; and (ii) an antigen, wherein said mucosal vaccine induces mucosal immunity; an agent suitable for prevention or treatment of allergy, comprising said antigen-drug vehicle and an allergen; the use of said mucosal vaccine in the preparation of a medicament for inducing mucosal immunity, wherein said mucosal vaccine is for administration into the nasal cavity or upper respiratory tract and the use of said agent in the preparation of a medicament for prevention or treatment of allergy, wherein said agent induces mucosal immunity. Preferably, the antigen-drug vehicle enables transmucosal and transdermal administration and the vaccine or agent for use in inducing mucosal immunity causes preferential and effective, particularly selective production of an antigen-specific secretory Immunoglobulin A. Also disclosed herein is a transmucosal or transdermal DDS, which is characterized by using the vehicle for desired antigen or drug.

Application or general use of the antigen-drug vehicle disclosed herein, brings general applications of a mucosal vaccine against various infections, an agent for prevention or treatment of allergy, and a transmucosal or transdermal DDS. The mucosal vaccine, which is an immunity means fit for the actual conditions of the natural infection, exerts remarkably excellent infection defense effects as compared to the conventional vaccines. In addition, the nasal cavity-mucosal IgA induced by the antigen-drug vehicle, brings inactivation of allergen, which makes reduced sensitization possible. Furthermore, application of aforesaid DDS for various drugs reinforces and promotes preventive and/or therapeutic effects of drugs by transmucosal and transdermal administration. As a result, this invention highly improves medical treatment, health and hygiene of the whole mankind, and also is hopeful good news to those engaged in the field of medical treatment, health and hygiene of the world. In addition, it provides widely means of imparting function and performance which makes it possible transmucosal and transdermal administration, which is simple compared to injection, widely for a biological preparation containing conventional and future vaccine, toxoid, and the like, and various drugs.

### Brief Description of the Drawings

Fig. 1 represents inhibition effects of influenza virus infection in (c) the nasal cavity and (d) the alveoli in (a) the cavity and (b) the alveoli of various nasal influenza vaccine administrations, and subcutaneous-injection influenza vaccine administration * represents the significance level from the group of vaccine-independent (without AD vehicle or adjuvant) administration by T-test (p<0.01). (Example 1)
Fig. 2 represents the amounts of IgA and IgG, which produces anti-influenza antibody in the nasal cavity-washing solution by influenza vaccine of (a) nasal administration and (b) subcutaneous injection. The white bar represents IgA amount, and the black bar represents IgG amount, respectively. (Example 2)
Fig. 3 represents the influence of the adjuvant on production of anti-influenza specific antibodies, IgA and IgG in the lung-washing solution by influenza vaccine of (a) nasal administration and (b) subcutaneous injection. (Example 3)
Fig. 4 represents the influence of PSF-2 and CTB on production of blood anti-influenza specific antibodies, IgA and IgG by influenza vaccine of (a) nasal administration and (b) subcutaneous injection. (Example 4)
Fig. 5 represents the influence of PSF-2 and CTB on the secretion level of TGF-β 1 in the mucosal membranes of (a) the nasal cavity and (b) the alveoli by nasal administration of influenza vaccine. (Example 6)
Fig. 6 represents the influence of PSF-2 and CTB on production of anti-influenza specific antibody in (a) the nasal cavity (b) the alveoli and (c) the blood by nasal administration of influenza vaccine. (Example 7)
Fig. 7 represents the influence of SPF-2 and CTB on production of various cytokines which is secreted from the nasal cavity, the alveoli and lymphocyte of the spleen by nasal administration of influenza vaccine.

### (Example 8)

Fig. 8 represents the influence of PSF-3 on production of anti-influenza specific antibody in (a) the nasal cavity, (b) the alveoli and (c) the blood by nasal administration of influenza vaccine. (Example 9)

### Best Mode for Carrying Out the Invention

Embodiments of the present invention will be explained below.
1. Explanation for terms and ingredients of the antigen-drug vehicle

### (1) Antigen-drug vehicle

The vehicle (Antigen-drug vehicle, hereinafter, simply referred to as the "AD vehicle" or "ADV") is a complex of lipid and protein which is designed to enable transmucosal administration and transdermal administration of antigen, drug, and the like. The AD vehicle comprises (a) to (c) below.
(a) Pulmonary surfactant protein B or its fragment (including not only natural fragment obtained by protease, but also artificial fragment obtained by genetic engineering or peptide synthesis, or variant fragment by substitution and/or deletion of at least one amino acid constituting such fragment).
(b) Pulmonary surfactant protein C or its fragment (including not only natural fragment obtained by protease, but also artificial fragment obtained by genetic engineering or peptide synthesis, or variant fragment by substitution and/or deletion of at least one amino acid constituting such fragment).
(c) Lipid such as phospholipid and fatty acid. The shape and the structure is a membrane form (lipid membrane of sheet form or rolling form) retaining polypeptide chain of thorn shape or spike shape, which is a shape wherein the ends of the hydrophobic area of the multiple polypeptide chains are intrusive into the lipid membrane to be implanted in spike shape, and different from conventional lipid vesicle (liposome).

If desired antigen, drug, and the like is subjected to coexistence, contact, capture, adhesion or binding (riding) with the antigen-drug vehicle disclosed herein, it makes it possible transmucosal administration and transdermal administration of such antigen, drug, and the like. In other words, aforesaid vehicle is a carrier of them, enabling transmucosal administration and transdermal administration of such antigen, drug, and the like.

In addition, ingredients of the AD vehicle, proteins, polypeptide, peptide or lipid used in preparation/production of the vehicle, i.e., pulmonary surfactant proteins B and C, a fragment thereof, a variant fragment by substitution and/or deletion of at least one amino acid constituting such fragment peptide, and the like, and lipid such as phospholipid, fatty acid, and the like will be explained in detail later.

### (2) Pulmonary surfactant

The pulmonary surfactant has been put to practical use from the middle 1990s in treatment of respiratory distress syndrome (RDS), and currently, various preparations derived from human, cattle, pig, and the like have been marketed widely to be in practical use (Non-Patent Document 2). In addition, synthetic peptide preparation containing active domain related to RDS treatment has been also marketed, and design development or synthesis of SP-B and SP-C analogues have been in progress (Non-Patent Document 3). Composition and constitution of the pulmonary surfactant are as follows: a complex comprising about 90 % of lipid (67.3% of phosphatidyl choline, 19.3% of phosphatidylglycerol, 3.2% of phosphatidyl serine, and other free fatty acids, and the like), and about 10% of protein (surfactant proteins A, B, C and D: Hereinafter, simply referred to as "SP-A", "SP-B", "SP-C" and "SP-D", respectively). The molecular weight is 28 to 36 kDa for SP-A, 15 kDa for SP-B, 3.5 kDa for SP-C and 43 kDa for SP-D. SP-A and SP-D are hydrophilic (water-soluble) and lectin-like (membrane-associated). SP-B and SP-C are hydrophobic (lipid-soluble) and lipid-bindable, and has intrusion ability into phospholipid membrane and surfactant action. The pulmonary surfactant protein genes derived from human, cattle, pig, and the like are known, and for example, the accession number of full-length base sequence of human SP-B gene DNA is J02761, and that of human SP-C (and SP-C1) is J03890 in GenBank/NCBI (http//www.ncbi.nlm.nih.gov./). The coding region (CDR) of the human SP-B and SP-C obtained from NCBI and the amino acid sequence encoded by them, are described below.
SEQ ID No. 1: CDR base sequence of human SP-B gene DNA;
SEQ ID No. 2: Human SP-B full-length amino acid sequence interpreted from SEQ ID No. 1;
SEQ ID No. 3: CDR base sequence of human SP-C gene DNA;
SEQ ID No. 4: Human SP-C full-length amino acid sequence interpreted from SEQ ID No. 3;
SEQ ID No. 5: CDR base sequence of SP-C1 located on human SP-C gene DNA; and
SEQ ID No. 6: Human SP-C1 full-length amino acid sequence interpreted from SEQ ID No. 5.

### (3) Protein or peptide used in this invention

In preparation/production of the antigen-drug vehicle related to the present invention, combination of SP-B and SP-C, and combination of SP-B and SP-C1, which are derived from mammals such as human, cattle, µg, whale, dolphin, and the like, or fishes such as tunny, shark, ray, yellowtail, and the like, can be used respectively. For example, use can be also made of combination of SP-B and SP-C and combination of SP-B and SP-C1, which are human-derived proteins comprising the full-length amino-acid sequence described in SEQ ID Nos. 2, 4 and 6, respectively, can be used respectively. Furthermore, for example, use can be also made of hydrophobic (lipid-soluble) region of SP-B and SP-C based on the hydrophobicity value of Kyte-Doolittle and a fragment comprising the region, a variant fragment by substitution and/or deletion of at least one amino acid constituting such fragment peptide. For example, use can be made of natural peptide or peptide obtained by genetic engineering or chemical synthesis comprising the amino acid sequence described in SEQ ID Nos. 7 to 20, long chain peptide from them comprising such peptide, and a variant or synthetic analogue by substitution and/or deletion of at least one amino acid constituting such peptide. The amino acid number is represented by the ordinal number in the order from Met occupying the N-end of each sequence as first amino acid, toward the C-end direction (direction from the left to the right of the described sequences).
SEQ ID No. 7: Amino acid sequence of No. 214 to No. 225 of SEQ ID No. 2 (SP-B fragment);
SEQ ID No. 8: Amino acid sequence of No. 257 to No. 266 of SEQ ID No. 2 (SP-B fragment);
SEQ ID No. 9: Amino acid sequence of No. 29 to No. 58 of SEQ ID Nos. 4 and 6 (SP-C fragment);
SEQ ID No. 10: Amino acid sequence of No. 1 to No. 20 of SEQ ID No. 2 (SP-B fragment);
SEQ ID No. 11: Amino acid sequence of No. 102 to No. 110 of SEQ ID No. 2 (SP-B fragment);
SEQ ID No. 12: Amino acid sequence of No. 119 to No. 127 of SEQ ID No. 2 (SP-B fragment);
SEQ ID No. 13: Amino acid sequence of No. 136 to No. 142 of SEQ ID No. 2 (SP-B fragment);
SEQ ID No. 14: Amino acid sequence of No. 171 to No. 185 of SEQ ID No. 2 (SP-B fragment);
SEQ ID No. 15: Amino acid sequence of No. 201 to No. 279 of SEQ ID No. 2 (SP-B fragment);
SEQ ID No. 16: Amino acid sequence of No. 253 to No. 278 of SEQ ID No. 2 (SP-B fragment);
SEQ ID No. 17: Amino acid sequence of No. 300 to No. 307 of SEQ ID No. 2 (SP-B fragment):
SEQ ID No. 18: Amino acid sequence of No. 317 to No. 330 of SEQ ID No. 2 (SP-B fragment);
SEQ ID No. 19: Amino acid sequence of No. 344 to No. 351 of SEQ ID No. 2 (SP-B fragment);
SEQ ID No. 20: Amino acid sequence of No. 358 to No. 381 of SEQ ID No. 2 (SP-B fragments):
SEQ ID No. 21: Amino acid sequence of No. 24 to No. 58 of SEQ ID Nos. 4 and 6 (SP-C fragment).

According to the present invention, use can be made of combination of at least one kind selected from a group consisting of SP-Bs comprising the amino acid sequences described in SEQ ID Nos. 2, 7, 8 and 10 to 20, and a fragment thereof, and at least one kind selected from a group consisting of SP-Cs (and SP-C1) comprising the amino acid sequences described in SEQ ID Nos. 4, 6, 9 and 21, and a fragment thereof.

### (4) Lipid used in the present Invention

As the phospholipid, phospholipid containing the pulmonary surfactant, for example, phosphatidyl choline (lecithin), dipalmitoyl phosphatidyl choline, phosphatidyl serine, and the like is preferably used. Additionally, use can be made of dipalmitoyl glycerophosphocholine, diacyl glycerophosphoglycerol, phosphatidylglycerol (cardiolipine), dilauroyl phosphatidylglycerol, dimiristoyl phosphatidylglycerol, dipalmitoyl phosphatidylglycerol, distearoyl phosphatidylglycerol, phosphatidyl inositol, phosphatidyl ethanolamine. phosphatidic acid, sphingomyelin, and the like. In addition, as the fatty acid, use can be made of lauric acid, myristic acid, palmitic acid, stearic acid, palmitoleic acid, oleic acid, and the like. In addition, use can be made of lipid derived from aquatic animal such as whale, tunny, dolphin, and the like, which has active pulmonary expansion.

### (5) The pulmonary surfactant preparations in the market for RDS treatment

According to the present invention, the pulmonary surfactant preparations in the market, for example, Surfacten (trademark), Infasurf, Curosurf, Humansurf, Exosurf, Alveofact, and the like can be used as the AD vehicle, which have been approved by the relevant authority for the safety and efficacy as a RDS treatment drug, and contains hydrophobic or lipid-soluble SP-B and SP-C and phospholipid. In addition to SP-B and SP-C, the marketed preparations containing hydrophilic or water-soluble SP-A and SP-D can be given to use after extracting the water-soluble proteins SP-A and SP-D with 1-butanol, and the like, and removing them to the detection limit or less. In addition, use of dry preparation is more desirable than liquid preparation considering the adjustment of the concentration to be used in preparation of the AD vehicle.

### 2. Novel findings as a base for the present invention

Under such very strict background techniques, this invention is made by keen observation and analysis of the present inventors from trial and error repeated over about 10 years, and profound knowledge and experience and novel idea, and it is based on the following astonishing findings.
(1) Conventional adjuvant reinforces antigen presenting-performance by causing inflammation, and originally there are four kinds of protein active ingredients, SP-A, SP-B, SP-C and SP-D, which are surfactants secreted by pulmonary or intestinal tract mucosal membrane. It has been found that if virus antigen is subjected to coexistence, contact, capture or adsorption with a complex of the combination of SP-B and SP-C excluding SP-A and SP-D, and phospholipid, or a complex of the combination of synthetic peptides of both fragments of SP-B and SP-C comprising lipid-soluble region thereof (active region), and lipid membrane (aforesaid AD vehicle), the antigen-presenting cells in the mucosal membrane of the nasal cavity are activated without causing inflammation, virus antigens are incorporated into the cells in good efficiency, and also anti-virus IgA production by the mucosal membrane is induced effectively and preferentially, and further selectively without causing induction of IgG production in the mucosal membrane or blood.
(2) In addition, it has been found that by adding to and mixing a complex of the combination of SP-B and SP-C, and phospholipid, or a complex of the combination of synthetic peptides of both fragments of SP-B and SP-C comprising lipid-soluble region thereof (active region), and lipid membrane (aforesaid AD vehicle), with influenza virus split antigen which is used as safe inactivated vaccine antigen conventionally, selective induction of secretory IgA production is achieved with the activation of the antigen-presenting cells sufficiently reinforced and supplemented, which is poor in independent split antigen compared with live vaccine, while maintaining high safety of the split antigen.

### 3. Process of completing the above-mentioned findings

(1) The present inventors have studied extensively to elucidate the mechanism of the development of influenza, and a treatment and prevention method for influenza. During the process, it has been elucidated that pulmonary surfactant adsorbs and inactivates tryptase clara of hemagglutinin (HA) processing protease of the respiratory tract, which manifests virus membrane infusion activity and infectious ability by restrictive cleavage of HA of the influenza virus membrane protein, resulting in inhibiting the growth cycle of the virus.
(2) As a result of continuous study, it has been found that in addition to the actions described above, the pulmonary surfactant selectively activates mucosal antigen-presenting cells, and thus activates immunity performance against virus antigen, leading to induction of secretory IgA, but not leading to induction of IgG. Furthermore, it has been shown that SP-B and SP-C are important as well as the lipid ingredient, as active ingredients reinforcing mucosal immunity in the pulmonary surfactant. The active region of these protein ingredients has been specified, and the efficacy of mucosal immunity reinforcement has been evaluated.
(3) Furthermore, studies have been progressed in view of a biological defense substance in the mucosal membrane of the respiratory tract, and defense against virus infection as described above, and it has been proved that the pulmonary surfactant, which is secreted in the living body, is involved in selective induction of IgA production as mucosal immunity adjuvant derived from the living body.
(4) Studies have continued giving attention to the fact that (a) the pulmonary surfactant, which is originally physiologically active substance in the living body, has property of adsorbing specific bio-substance (Kido H., et al. FEBS Lett. Pulmonary surfactant is a potential endogenous inhibitor of proteolytic activation of Sendai virus and influenza A virus, 322 (29), 115-119, 1992), (b) the pulmonary surfactant is secreted from alveolar type II cell or clara cell, and then selectively incorporated into macrophage (Akira Suwabe, J. Jpn. Med. Soc. Biol. Interface; Surfactant metabolism disorder in alveolar proteinemia, 33, 10-13, 2002), and (c) the pulmonary surfactant is incorporated and metabolized in analogous cells, for example, antigen-presenting cells (dentritic cells).
   As a result, it has been found that only SP-B and SP-C among the protein ingredients, and lipid ingredients of the pulmonary surfactant, function as the "AD vehicle" of mucosal vaccine which selectively induces IgA production, and that the active ingredient region of SP-B or active domain of mucosal immunity induction is a peptide comprising the following amino acid sequences:
   SP-B 214-225: Leu Ile Lys Arg Ile Gln Ala Met Ile Pro Lys Gly (SEQ ID No. 7); and
   SP-B 257-266: Leu Leu Asp Thr Leu Leu Gly Arg Met Leu (SEQ ID No. 8).

   In addition, it has been found that the active ingredient region of SP-C or active domain of mucosal immunity induction is a peptide comprising the following amino acid sequences:
(5) In addition, it has been found as the mechanism of selective IgA induction that the active ingredients of the pulmonary surfactant effectively conduct antigen presentation of T-lymphocyte by inducing increased expression of MHC Class II, CD40 and B7-2 of antigen-presenting dendritic cells, and in addition, promote class switch to IgA-producing B-lymphocyte by inducing cytokine TGF-β1 of a local mucosal membrane.

### 4. The objects of the present invention completed based on the findings and the details described above are as follows:

(1) The first object is to establish a mucosal immunity method. By providing the "AD vehicle" and using it, the present invention achieves selective induction of production of antigen-specific IgA, which is an active substance of mucosal immunity, and also establishes induction of safe and effective (with no adverse effects) mucosal immunity, and a method thereof.
(2) The second object is to improve qualities related to safety, efficacy and uniformity of the AD vehicle by use of a synthetic peptide. Disclosed herein is a complex (the AD vehicle), which is prepared with a synthetic peptide of active domain of SP-B mucosal immunity induction (comprising each amino acid sequence of SP-B 214-225 and SP-B 257-266 described above), a synthetic peptide of active domain of SP-C mucosal immunity induction (comprising each amino acid sequence of SP-C 29-58 described above), a synthetic analogue thereof, a long-chain synthetic peptide comprising these amino acid sequence as a part, and the like, and a lipid ingredient of the pulmonary surfactant, and improves the quality of the AD vehicle.
(3) The third object is conversion of conventional subcutaneous inoculation of vaccine to mucosal administration. The present invention employs the AD vehicle in inactivated vaccine of respiratory tract-infection virus, for example, inactivated vaccine of influenza, SARS, the measles, rubella, mumps, and the like, and inactivated vaccine of intestinal tract-infection virus, for example, inactivated vaccine of rotavirus, poliovirus, and the like, and converts subcutaneous inoculation vaccine thereof to mucosal vaccine.
(4) The fourth object is to provide a method of using the AD vehicle in inactivated vaccine against virus infection via mucosal membrane except for the respiratory tract and the intestinal tract, for example, inactivated vaccine against AIDS, Type B hepatitis. Type C hepatitis, and the like.
(5) The fifth object is to provide a method of using the AD vehicle in DNA vaccine, live vaccine, prevention and treatment of allergy, and the like.
(6) The sixth object is to provide a method of using the AD vehicle in transdermal inoculation (application, pasting, and the like) as an immunity route which can induce IgA besides the mucosal membrane.
(7) The seventh object is to open a way of use and application of the AD vehicle in agriculture, fishery, and the like, not only in DDS or pharmaceutics.

The AD vehicle used herein, has different performances and actions from adjuvants which have been used conventionally in immunology as follows: Specifically, conventional adjuvants are inoculated usually subcutaneously or intramuscularly, causing local inflammation reaction and attracting antigen-presenting cells or B- and T-lymphocytes, and has a foreign substance exerting its ability as active ingredient. In addition, they are used in combination with mineral oil or metal salt causing sustained-release and retention of antigen to maintain inflammation reaction over long time. In addition, those known as conventional mucosal vaccine/adjuvants are as described above, and a foreign substance such as Escherichia coli heat-labile live toxin and cholera toxin, and has risk of causing harmful actions or adverse actions. On the contrary, the AD vehicle used herein causes no local inflammation reaction. In addition, the AD vehicle is derived from biological ingredient, and on top of that, it is limited to active ingredient in the pulmonary surfactant or an active domain thereof, and uses low-molecular-weight peptide comprising such domain or domain region to achieve effective mucosal vaccine. Accordingly, the AD vehicle is very safe and non-invasive.

### 5. The following (1) to (5) are disclosed herein or provided by the present invention:

(1) Disclosed herein is an antigen-drug vehicle, which is a complex comprising at least one fragment selected from pulmonary surfactant protein B or multiple fragments derived from the protein B, at least one fragment selected from pulmonary surfactant protein C or multiple fragments derived from the protein C, and at least one lipid.
   More specifically, the AD vehicle is a complex comprising at least three substances in total, which are selected by at least one kind from each group of following Group I (group of pulmonary surfactant protein B, and natural and synthetic polypeptide derived or originated from the protein B), Group II (group of pulmonary surfactant protein C, and natural and synthetic polypeptide derived or originated from the protein C) and Group III (group of lipid such as phospholipid, fatty acid, and the like).
   (Group I) Pulmonary surfactant protein B and a polypeptide comprising following amino acid sequence described in SEQ ID No. 2 (The amino acid number is assigned in the order from Met occupying the N-end of each sequence as first amino acid, toward the C-end): Nos. 1 to 381 (SEQ ID No. 2), Nos. 214 to 225 (SEQ ID No. 7), Nos. 257 to 266 (SEQ ID No. 8), Nos. 1 to 20 (SEQ ID No. 10), Nos. 102 to 110 (SEQ ID No. 11), Nos. 119 to 127 (SEQ ID No. 12), Nos. 136 t 142 (SEQ ID No. 13), Nos. 171-185 (SEQ ID No. 14), Nos. 201-279 (SEQ ID No. 15), Nos. 253-278 (SEQ ID No. 16), Nos. 300-307 (SEQ ID No. 17), Nos. 317-330 (SEQ ID No. 18). Nos. 344-351 (SEQ ID No. 19), NOs. 358-381 (SEQ ID No. 20), a polypeptide comprising at least one sequence of the above-mentioned amino acid sequences as an active domain, a polypeptide by substitution and/or deletion of at least one amino acid in the above-mentioned amino acid sequences, a synthetic analogue thereof, a modified body thereof by saccharide or saccharide chain, and the like.
   [Group II] Pulmonary surfactant protein C and a polypeptide comprising following amino acid sequence described in SEQ ID No. 4 (The amino acid number is assigned in the order from Met occupying the N-end of each sequence as first amino acid, toward the C-end): Nos. 1 to 197 (SEQ ID No. 4), Nos. 29 to 58 (SEQ ID No. 9), Nos. 24 to 58 (SEQ ID No. 21), a polypeptide comprising the amino acid sequence of Nos. 1 to 191 of SEQ ID No. 6, a polypeptide comprising at least one sequence of the above-mentioned amino acid sequences as an active domain, a polypeptide by substitution and/or deletion of at least one amino acid in the above-mentioned amino acid sequences, a synthetic analogue thereof, a modified body thereof by saccharide or saccharide chain, and the like.
   [Group III] Lipid such as phospholipid like phosphatidyl choline, dipalmitoyl phosphatidyl choline, phosphatidyl serine, dipalmitoyl glycerophosphocholine, diacyl glycerophosphoglycerol, phosphatidylglycerol, phosphatidyl inositol, phosphatidyl ethanolamine, phosphatidic acid, and the like, fatty acid like lauric acid, myristic acid, palmitic acid, stearic acid, oleic acid, and the like, and the like.

   In addition, the antigen-drug vehicle preferably has a shape and structure wherein the above-mentioned Group III is a lipid membrane of sheet form or rolling form, and multiple chains of each of the above-mentioned Group I and Group II are implanted in spike shape with the hydrophobic area ends being intrusive into the lipid membrane.
(2) The present invention provides a mucosal vaccine of claim 1.
(3) The present invention provides an agent suitable for prevention and treatment of allergy of claim 6. The action and effects are by deactivation or reduced sensitization of allergen such as cedar pollen coming by air and sucked, tick, and the like, by mucosal IgA in the nasal cavity or the nasal pharynx.
(4) Also disclosed herein is a transmucosal and/or transdermal DDS which is obtained by subjecting the antigen-drug vehicle of the above-mentioned (1) to coexistence, contact, capture or adsorption with a drug in an effective amount.
(5) The present invention provides the mucosal vaccine or agent of the above-mentioned (2) or (3) for use in inducing mucosal immunity or for prevention or treatment of allergy. The present invention provides the use of a mucosal vaccine of the above-mentioned (2) in the preparation of a medicament for inducing mucosal immunity, wherein said mucosal vaccine is for administration into the nasal cavity or upper respiratory tract. The present invention also provides the use of an agent of the above-mentioned (3) in the preparation of a medicament for prevention or treatment of allergy, wherein said agent induces mucosal immunity. Also disclosed herein is a method of inducing mucosal immunity which is characterized by administering a mucosal vaccine, which is obtained by subjecting the antigen-drug vehicle of the above-mentioned (1) to coexistence, contact, capture or adsorption with antigen, into the nose or upper respiratory tract.

The induction of mucosal immunity in the inventions of the above-mentioned (2), (3) and (5) is preferably characterized by promoting the production of an IgA antibody in a local mucosal membrane, and further by promoting the production of TGF-β1 and Th2-type cytokine in the local mucosal membrane.

### 6. Hereinafter, the composition and preparation of the AD vehicle, the preparation of mucosal vaccine, and the preparation of the transmucosal and transdermal DDS will be explained.

### (1) Composition of the AD vehicle

Followings are dry wt.% of the three groups of the above-mentioned Group I (group of pulmonary surfactant protein B, and natural and synthetic polypeptide derived or originated from the protein B), Group II (group of pulmonary surfactant protein C, and natural and synthetic polypeptide derived or originated from the protein C) and Group III (group of lipid such as phospholipid, fatty acid, and the like): about 0.1 to about 6.0 wt.% of Group I, about 0.1 to about 6.0 wt.% of Group II and about 88 to about 99.8 wt.% of Group IIII. In preparation of the antigen-drug vehicle, the composition is adjusted to Group I% + Group II% + Group III%=100% by wt.%.

### (2) Preparation of the AD vehicle

Preparation procedures are as follows, for example: 2 mg of Group I, 2 mg of Group II and 96 mg of Group III are weighed, respectively (Group I% + Group II% + Group III%=100% by wt.%), which are suspended uniformly in 5 ml isotonic solution, for example, physiological saline solution or phosphate buffered saline (PBS). Resultant suspension is given to use as a solution of the antigen-drug vehicle (100 mg/5 ml). The vehicle is prepared in each use. For suspension, ultrasonic wave, homogenizer, mixer, shaker, and the like can be used. The ultrasonic wave is likely to cause liquid change (increase of viscosity) by excessive treatment, and thus, a mixer, for example, box mixer (e.g., Vortex mixer (trademark)) is preferably used.

For specification of 96 mg of the lipid of Group III, for example, a mixture of 71 mg of phosphatidyl choline, 21 mg of phosphatidylglycerol and 4 mg of phosphatidyl serine, and the like can be adopted (the total of the lipid amount is 96 mg). In addition.. in case of using marketed pulmonary surfactant preparation for RDS treatment, which is ensured to contain SP-B and SP-C excluding SP-A and SP-D, the suspension prepared in accordance to the instruction manual, can be given to use as it is or as a solution of the antigen-drug vehicle.

### (3) Preparation of mucosal vaccine

A mucosal vaccine is prepared by adding to and mixing the solution of the antigen-drug vehicle with an undiluted vaccine solution that the dry-weight ratio of the amount of the antigen-drug vehicle (V) to the amount of antigen in the vaccine (A) becomes about 0.2 to about 5. For example, if the antigen content is 1 µg/ml in 1,000 ml of the vaccine undiluted solution, and the weight ratio adopted is A/V=1, the addition and mixing amount of the solution of the antigen-drug vehicle (100 mg/5 ml), which has been prepared in the above-mentioned (2), is 50 µl. For uniform mixing, homogenizer, mixer, shaker, stirrer, and the like can be used.

### (4) Preparation of transmucosal or transdermal DDS

The transmucosal or transdermal DDS can be prepared by using a drug instead of an antigen in the same manner as in the above-mentioned (3).

### Examples

Hereinafter, the constitution and effects of the present invention will be explained in detail by showing Examples. However, this invention is not limited only to these examples, explanation and description.

Raw materials, experimental procedures, and the like used in these examples are as follows.

### (1) Pulmonary surfactant

The pulmonary surfactant used as the "antigen-drug vehicle (the AD vehicle) is a sample prepared from bovine lung by the method of Howgood, et al. (PSF-1) (Howgood S, et al.,: Effects of a surfactant-associated protein and calcium ions on the structure and surface activity of lung surfactant lipids. Biochemistry, 24, 184-190, 1985), or largely a sample prepared by extracting the former sample with 1-butanol to remove water-soluble protein ingredients. SP-A and SP-D, or reduce them to detection limit or less (PSF-2) (Haagasman HP, et al.,: The major lung surfactant protein, SP28-36, is a calcium-dependent, carbohydrate binding protein, J. Biol. Chem. 262, 13977-13880, 1987), and further any synthetic peptide of the active regions of the lipid-soluble proteins, SP-B or SP-C, which contains 40 wt.% or more to the whole of phospholipid comprising mainly phosphatidyl choline and dipalmitoyl phosphatidyl choline, and further contains 10 to 20% of phosphatidylglycerol and 2 to 5% of phosphatidyl serine, which are similar to the pulmonary surfactant lipid, or a sample containing 0 to 3.5% of both peptides in combination. Among these samples, investigated were a sample containing both peptides in combination of the active regions of SP-B and SP-C (PSF-3), a sample containing a synthetic peptide of the active region of SP-B (PSF-4) and a sample containing a synthetic peptide of the active region of SP-C (PSF-5). Alternatively, known samples corresponding to PSF-1 or PSF-2, for example, Survanta, Infasurf, Curosurf, Humansurf, Exosurf, Alveofact, and the like (trademark) are also used.

### (2) Animal

6 week-old female BALB/c mouse, and 10 week-old Hartley guinea pig were purchased from Japan SLC, Inc. (Shizuoka, Japan) and used. All of the animal experiments were conducted in the cage for infected animals (P2 level) of the experimental animal center of the medical faculty of University of Tokushima, and conducted in accordance of the guideline of the animal experiment committee of the medical faculty of University of Tokushima.

### (3) Preparation of split-type influenza vaccine

Preparation of split-type influenza vaccine was conducted with the following procedures using the suspension derived from a grown egg where the influenza virus, A Aichi/68/2/H3N2 strain was inoculated (1 × 10⁸ Plaque forming unit [PFU]) (supplied by Professor Masanobu Ouchi in microbiology class of Kawasaki medical school). β-propiolactone (Wako Pure Chemical Industries. Ltd., Osaka, Japan) was added to the virus suspension, which was dialyzed in 0.004 M PBS (TakaraBio, Inc., Shiga. Tokyo, Japan) overnight, to 0.05% of the liquid amount and 8 nM of the final concentration, and incubated for 18 hours in an ice bath. Then, it was incubated for 1.5 hours at 37°C to hydrolyze the β-propiolactone. Then, Tween 20 (Wako Pure Chemical Industries, Ltd.) was added to 0.1% of the final concentration, and diethylether (Wako Pure Chemical Industries, Ltd.) was further added in the equal amount to that of Tween, and mixed with shaking for two hours at 4°C. This solution was centrifuged at 2,000 rpm for 5 minutes to collect the aqueous layer. Furthermore, removal of diethylether was conducted from the aqueous layer with Automatic Environmental SpeedVac System (SAVANT INSTRUMENTS, INC., New York. USA). This was filtered with a 0.45 µm filter (MILLIPORE, Massachusetts, USA), to prepare inactivated split-type influenza vaccine.

### (4) Immunization

To the split-type influenza vaccine prepared by the production method described above were mixed and used the above-mentioned pulmonary surfactant (PSF-1), 1-butanol extracted pulmonary surfactant (PSF-2), the synthetic peptides of the active regions of SP-B and SP-C and the pulmonary surfactant lipid (PSF-3, -4 and -5), known pulmonary surfactant product, or cholera toxin B subunit (CTB, SIGMA, Missouri, USA) as an immune adjuvant. The pulmonary surfactant or the corresponding sample described above was suspended in PBS in situ at a concentration required in vaccine administration, and treated with ultrasonic wave for 5 minutes at room temperature to give a uniform suspension. To this was added 0.1 µg of the split-type influenza vaccine to 0.1 µg by dry weight of the pulmonary surfactant or the corresponding sample, and mixed in a vortex mixer, and left for 1 hour at room temperature to be used. CTB was adjusted in situ in the similar manner, and mixed in 0.1 µg to 0.1 µg of the split-type influenza vaccine (Watanabe I, et al.,: Characterization of protective immune responses induced by nasal influenza vaccine containing mutant cholera toxin as a safe Adjuvant (CT112K). Vaccine 2002; 20: 3443-55).

For nasal administration of the vaccine, the above-mentioned adjusted product was diluted in PBS to be a 0.1 µg/l µl solution of Phosphate buffered saline (PBS), and this was administered to both sides by 1 µl, respectively per one animal and 2 µl in total was nasal-administered to both sides of the nasal cavities of the mouse anesthetized with ether. For conventional subcutaneous injection, which was conducted for comparison, the split-type influenza vaccine was diluted in PBS to be a solution of 0.1 µg/50 µl, and this was administered to the hypoderma of the mouse neck. For the control group, PBS was administered in the same amount as that of the vaccine solution. After 4 weeks, second immunization was carried out in the same manner as the first immunization, and two weeks after the second immunization, the washing solutions of the nasal cavity and the alveoli, and the serum of the mouse, were prepared, which were used in measurement of virus-specific IgA and IgG, and virus infection experiment.

### (5) Preparation of the washing solutions of the nasal cavity and the alveoli, and the serum of the mouse

The vaccine-administered mouse was cut open in the abdomen and the chest under pentobarbital anesthesia, and the trachea was cut open, and Atom venous catheter tapered 3 Fr (Atom medical Corporation, Tokyo, Japan) was inserted into the lung, and 1 ml of physiological saline solution was infused, and this solution was collected. This was repeated three times, and the collected solution. 3 ml in total was used as the washing solution of the alveoli. After collecting the washing solution of the alveoli, the atom vein catheter was inserted from the opened trachea to the direction of the nasal cavity, and 1 ml of physiological saline solution was infused, and the solution coming from the nose was collected. This solution was used as the washing solution of the nose. Furthermore, blood collecting was conducted from the heart, and the serum was prepared by centrifuge at 5,000 rpm for 10 minutes.

### (6) Quantitization of protein

The protein content in the washing solutions of the nose and the lung, and the serum was measured with BCA Protein Assay Reagent Kit (PIERCE, Illinois, USA) (Smith PK., et al.,: Measurement of protein using bicinchoninic acid. Anal. Biochem., 150, 76-85, 1985). Absorbance at 562 nm was measured with SPECTRAmax PLUS 384 (Molecular Devices Corporation, California. USA).

### (7) Experiment for virus infection and evaluation for infection value.

The influenza virus strain. A Aichi/68/2/H3N2 strain, which is the same as the virus strain used in the preparation of the split-type influenza vaccine, was used in infection. Two weeks after completion of the second immunization, the mouse was anesthetized with ether, and a suspension derived from an egg where the influenza virus was grown, was dropped for infection into both sides of the nasal cavities in 7 × 10⁴ PFU/3 µl. 3 weeks after the infection, the washing solutions of the nasal cavity and the alveoli were prepared in the same manner as described above, which were used in evaluation of virus infection value. The evaluation of virus infection value was conducted using A 549 cells (supplied by Professor Masanobu Ouchi in microbiology class of Kawasaki medical school). A 549 cells were incubated under the condition of 5% bovine fetal serum/DMEM (Gibco, New York, USA). A 549 cells were subcultured to be 100% confluent in 6 well-incubation plate (Greiner Deutsche Stuttgart), and changed to no-serum medium after 24 hours. The washing solutions of the nasal cavity and the alveoli of influenza infection mouse were dropped in 500 µl, respectively to each well, and incubated in CO₂ incubator at 37°C for 12 hours to 16 hours. To this was added the 1% PBS solution of erythrocyte, which was collected from the guinea pig, and the mixture was left for 5 minutes at room temperature. The mixture was washed with 1 mM Ca²⁺/Mg²⁺ PBS, and evaluation of virus infection value was conducted by counting the cell agglutinating erythrocyte as a virus infection cell (Tashiro M., Homma M.: Pneumotropism of Sendai virus in relation to protease-mediated activation in mouse lungs. Infect. Immun. 39, 879-888, 1983).

### (8) Purification of anti-influenza specific IgA and IgG

Purification of anti-influenza specific IgA and IgG was conducted as follows to be used as standard for quantitization in ELISA assay. IgG fractions were purified from the lung-washing solutions of the influenza vaccine-administered and virus-infected mice by affinity chromatography with recombinant E. coli expression Protein G cephalose 4B column (ZYMED LABORTORIES INC, San Francisco, USA). Anti-mouse IgA goat IgG (SIGMA) was bound to BrCN-activated cephalose 4B column (Amersham Bioscience, New Jersey, USA), and using this, IgA fractions were purified by affinity chromatography from the Protein G passing-through fraction. To purify virus-specific antibody from these IgG and IgA fractions, inactivated split-type influenza vaccine used in immunization was bound to BrCN activated cephalose column, and anti-influenza specific IgA and IgG were purified, respectively by antigen affinity chromatography from the IgG and IgA fractions using this. Coupling of the split-type influenza protein as a ligand to the column was carried out by the binding reaction with 0.1 M NaHCO₃/0.5 M NaCl buffer solution (pH 8.5), and removing the free ligand with 0.1 M acetic acid/0.5 M NaCl buffer solution (pH 8.5) and neutralizing by PBS (pH 7.5). After affinity-binding reaction by PBS (pH 7.5) and removal of free antibody, each of the affinity chromatography was subjected to elution of the specific antibody by glycine-HCl buffer solution (pH 2.8). Eluted fractions were neutralized immediately by 0.5 M Tris HCl buffer solution (pH 9.0), and dialyzed with MilliQ water and lyophilized, which was used as dissolved in PBS in situ.

### (9) Quantitization of anti-influenza antibody

Contents of anti-influenza IgA and IgG in the washing solutions of the nasal cavity and the alveoli, and the serum, were quantitized by ELISA assay. The ELISA assay was carried out according to the method of Mouse ELISA Quantization kit of BETHYL LABORATORIES (Texas, USA). To each well of 96 well Nunc immunoplate (Nalgen Nunc International, New York, USA), 1 µg of the vaccine and 100 µl of 1 µg/ml PBS solution of bovine serum albumin (BSA, SIGMA, Missouri, USA) were added, and reaction for layer-solidification was conducted at 4°C overnight. Then, each well was washed three times with the washing solution (50 mM Tris, 0.14 M NaCl, 0.05% Tween 20, pH 8.0) to remove the vaccine solution. To each well was added 50 mM Tris-HCl buffer solution containing 0.15 M NaCl and 1% BSA, and the blocking reaction was carried out at room temperature for 1 hour. Each well was washed three times with the washing solution, and then added were 100 µl of the washing solutions of the nasal cavity and the alveoli, and the serum, which had been diluted with a suitable amount of a sample-binding buffer solution (50 mM Tris, 0.15 M NaCl, 1% BSA. 0.05% Tween 20, pH 8.0), and the mixture was left for reaction at room temperature for 2 hours. Color reaction was conducted with TMB Microwell Peroxidase Substrate System (Kirkegaard & Perry Laboratories, Inc. Maryland, USA), using Goat anti-mouse IgA or IgG-horse rADish peroxidase (HRP) (BETHYL LABORATORIES INC.) as secondary antibody. The reaction was terminated by adding 100 µl of 2 M H₂SO₄ (Wako Pure Chemical Industries, Ltd.) to each well, and absorbance at 450 nm was measured with SPECTRAmax PLUS 384. Anti-influenza 10 ng of IgA and IgG purified from the above-mentioned lung-washing solution was treated in the same manner as a standard for quantitization, and obtained absorbance was used.

### (10) Preparation of dendritic cells and flow cytometry

Preparation of dendritic cells was carried out from the nose, lung and spleen collected from the mice of each group (4 in one group) 2 days after the first immunization, by the method of Gonzalez-Juarrero M (Gonzalez-Juarrero M, Orme 1M.: Characterization of murine lung dendritic cells infected with Mycobacterium tuberculosis. Infect Immun. 2001; 69: 1127-33). Preparation of dendritic cells from the nose and collagenase treatment therefor was conduced according to the method of Asanuma H, et al. (Asanuma H, et al.,: Characterization of mouse nasal lymphocytes isolated by enzymatic extraction with collagenase. J. Immunol. Methods 1995: 187: 41-51). The dendritic cells prepared from each of the tissues, were washed with 1 mM EDTA/PBS, and added per 10⁶ cells were each 1 µg/ml of FITC conjugated Anti-IA/IE (MHC class II) and PE conjugated Anti CD40 or FITC conjugated Anti-CD80 (B7-1) and PE conjugated Anti CD86 (B7-2) (BD Bioscience, New Jersey, USA), and left for reaction for 30 minutes on the ice as 50 µl 1 mM EDTA/PBS suspension. Free antibody was removed by conducting washing twice with 1 ml of 1 mM EDTA/PBS, and the cells were resuspended in 1 ml of 1 mM EDTA/PBS. Using this, detection for the modification factor on the cell surface was conducted by BD FACS Callibur (BD Bioscience).

### (11) Quantization of TGF-β1

Secretion amount of TGF-β1 in the washing solutions of the nasal cavity and the alveoli was quantitized by ELISA assay. The ELISA assay was conducted using TGF-β1 ELISA kit (BIOSOURCE INTERNATIONAL, California, USA) according to the instruction attached to the kit.

### (12) Quantitization of various cytokines

The amounts of cytokines secreted, respectively in the washing solutions of the nasal cavity and the alveoli and from the lymphocytes of the spleen (Interleukin 4: IL-4, IL-5. IL-6 and IL-13), were quantitized by marketed ELISA kit.

### (13) Preparation of PSF-3 comprising synthetic peptides of SP-B and SP-C and phospholipid

Each peptide of SP-B 253-278 (SEQ ID No. 16) and SP-C 24-58 (SEQ ID No. 21) was chemically synthesized by known method. These peptides were added to a phospholipid membrane (dipalmitoyl phosphatidyl choline (75), phosphatidylglycerol (25) and palmitic acid (10)) to prepare a phospholipid membrane of plate form, and AD vehicle PSF-3 was prepared.

### EXAMPLE 1

### Comparison of nasal influenza vaccine and subcutaneous-injection influenza vaccine on virus growth inhibition action

As the nasal vaccine, 0.1 µg of the split-type influenza vaccine was administered to both noses of BALB/c mouse by 1 µl, respectively as a PBS solution, alone or with 0.1 µg of 1-butanol-extracted (excluding SP-A and SP-D) surfactant (PSF-2 below) as the "AD vehicle", or 0.1 µg of CTB. As the subcutaneous-injection vaccine, the vaccine in the same amount as that of the nasal vaccine was administered as 50 µl PBS solution in total to the hypoderma of the neck of BALB/c mouse, alone or with adding PSF-2 as the AD vehicle or CTB as an adjuvant. After 4 weeks, second immunization was conducted in the same manner as in the first immunization. To the control group, same amount of PBS was administered, respectively. 2 weeks after the second immunization, influenza virus of 6.6 × 10⁴ PFU was subjected to nasal infection as 3 µl PBS solution. 3 days after the infection, the mouse was slaughtered, and the washing solutions of the nasal cavity and the alveoli were prepared, and using this, evaluation of virus infection value was conducted (n=15-20; average±SE; *, the significance level by T-test was p<0.01 to the vaccine administration group)

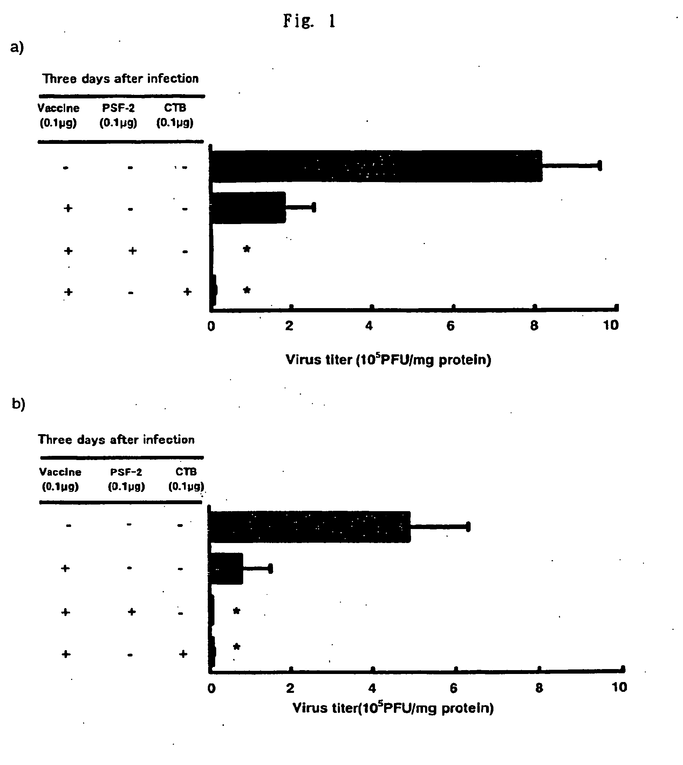

As shown in Figs. 1 (a) and (b), in case of nasal influenza vaccine administration, growth of the influenza virus in the washing solutions of the nasal cavity and the alveoli was inhibited even by the independent vaccine administration, but PSF-2 or CTB reinforced the effects significantly and growth of the influenza was nearly perfectly inhibited, ensuring the effects of the vaccine. Though not shown in the figure, in the case of using PSF-1 or PSF-3 instead of PSF-2, similar effects were obtained. Similar phenomena were also found for PSF-4 and -5, but the effects were attenuated.

Though not shown in the figure, even in case that PSF-2 or CTB were nasally administered as independent, respectively in the first immunization and the second immunization, inhibitory effects for virus growth were not found in either case, and thus the effects of PSF-2 or CTB were determined as reinforcement effects for the vaccine effects.

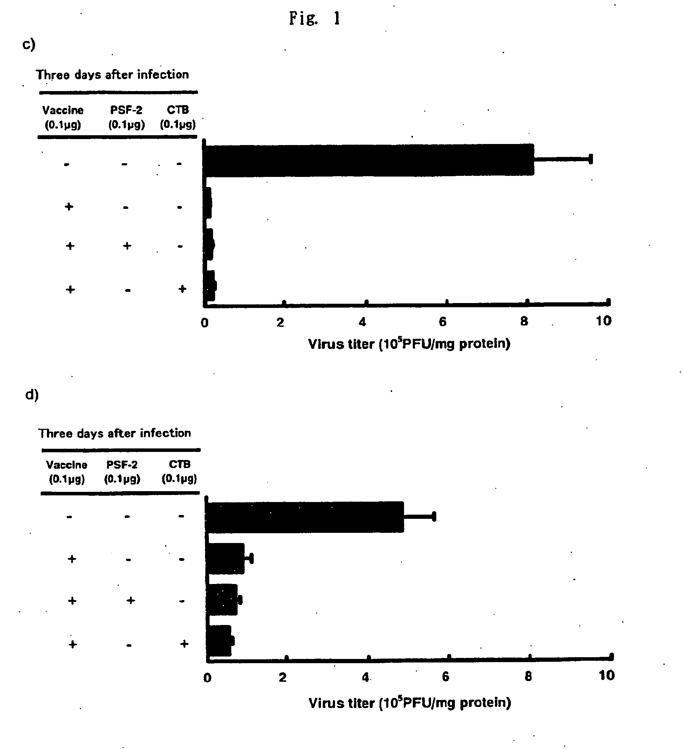

On the other hand, in case of subcutaneous-injection influenza vaccine, as shown in (c) and (d) of Fig. 1, the titer (PFU) of the influenza virus in the washing solutions of the nasal cavity and the alveoli was reduced significantly even with the independent vaccine, so the effects of the vaccine was found, but reinforcement effects of PSF-2 or CTB were not found. In other words, in case of subcutaneous administration, nearly no or very little reinforcement effects of PSF-2 or CTB on immunity were found. Though not shown in the figure in this experiment, even in case that PSF-2 or CTB is subcutaneously administered as independent, respectively in the first immunization and the second immunization, inhibitory effects for virus growth were not found in either case. Though not shown in the figure, in the case of using PSF-1, PSF-3. PSF-4 and PSF-5 instead of PSF-2, reinforcement effects on the vaccine action were not found.

### EXAMPLE 2

### Influence of PSF-2 or CTB. on production of anti-influenza specific antibodies (IgA and IgG) in the washing solution of the nasal cavity after (a) nasal and (b) subcutaneous-injection influenza vaccine administration

In the same manner as described in Fig. 1, as the nasal vaccine, 0.1 µg of the split-type influenza vaccine was administered to both noses of BALB/c mouse by 1 µl, respectively, i.e., 2 µl in total as a PBS solution, alone or with 0.1 µg of PSF-2 as the "AD vehicle", or 0.1 µg of CTB as an adjuvant. As the subcutaneous vaccine, the vaccine in the same amount as that of the nasal vaccine. PSF-2 or CTB was administered as 50 µl PBS solution to the hypoderma of the neck of BALB/c mouse. After 4 weeks, second immunization was conducted in the same manner as in the first immunization. To the control group, same amount of PBS was administered, respectively. 2 weeks after the second immunization, influenza virus of 6.6 × 10⁴ PFU was subjected to nasal infection as 3 µl PBS solution. 3 days after the infection, the mouse was slaughtered, and the washing solution of the nasal cavity was prepared, and using this, evaluation of virus infection value was conducted (n= 5-20; average±SE:*, the significance level by T-test was p<0.01 to the vaccine administration group).

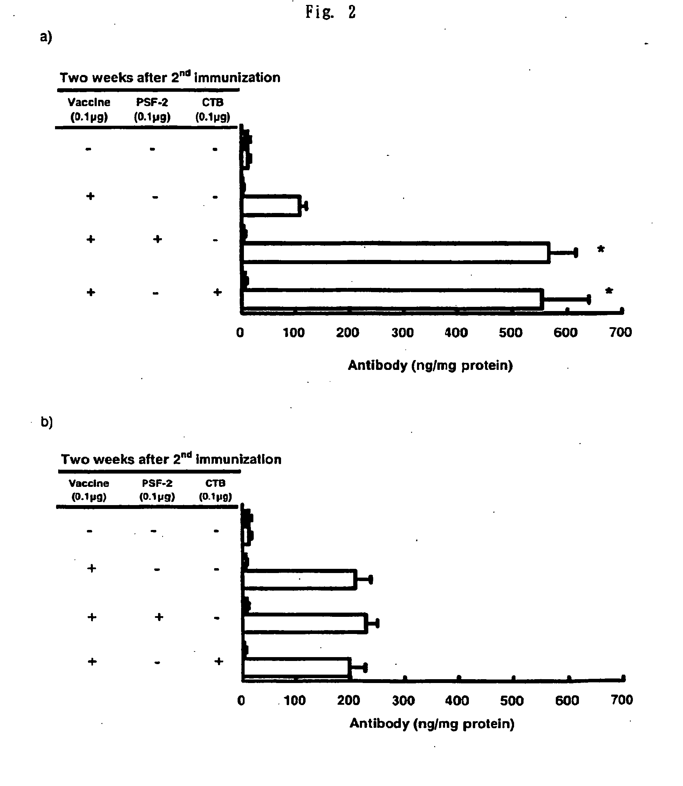

The results are shown in Figs. 2 (a) and (b). By the split-type influenza vaccine nasally administered, anti-influenza specific IgA was selectively produced in the nasal cavity, and thus increased in the washing solution, but PSF-2 or CTB increased the amount of this specific IgA in equivalent extent remarkably. On the other hand, even in case of the subcutaneous injection, increase of specific IgA amount in the washing solution of the nasal cavity by independent split-type influenza vaccine was found, but the extent was low compared to that of the nasal administration. In addition, in case of the subcutaneous injection of the vaccine, immunity reinforcement effects of PSF-2 or CTB were not found in either case in production of IgA or IgG. Though not shown in the figure, in the case of using PSF-1 or PSF-3 instead of PSF-2, similar effects were obtained. Similar phenomena were also found for PSF-4 and -5, but the effects were attenuated.

### EXAMPLE 3'

### Influence of PSF-2 or CTB on production of anti-influenza specific antibodies (IgA and IgG) in the washing solution of the lung by influenza vaccine of (a) nasal administration and (b) subcutaneous-injection

As the nasal vaccine, 0.1 µg of the split-type influenza vaccine was administered to both noses of BALB/c mouse by 1 µl, respectively, i.e., 2 µl in total as a PBS solution, alone or with 0.1 µg of PSF-2 as the "AD vehicle", or 0.1 µg of CTB as an adjuvant. As the subcutaneous vaccine, the vaccine in the same amount as that of the nasal vaccine, PSF-2 or CTB was administered as 50 µl PBS solution to the hypoderma of the neck of BALB/c mouse. After 4 weeks, second immunization was conducted in the same manner as in the first immunization. To the control group, same amount of PBS was administered, respectively. 2 weeks after the second Immunization, influenza virus of 6.6 × 10⁴ PFU was subjected to nasal infection as 3 µl PBS solution. 3 days after the infection, the mouse was slaughtered, and the washing solution of the lung was prepared, and using this, influence of the antigen-drug vehicle on production of anti-influenza specific antibodies (IgA and IgG) was investigated (n=15-20: average±SE; *, the significance level by T-test was p<0.01 to the vaccine administration group).

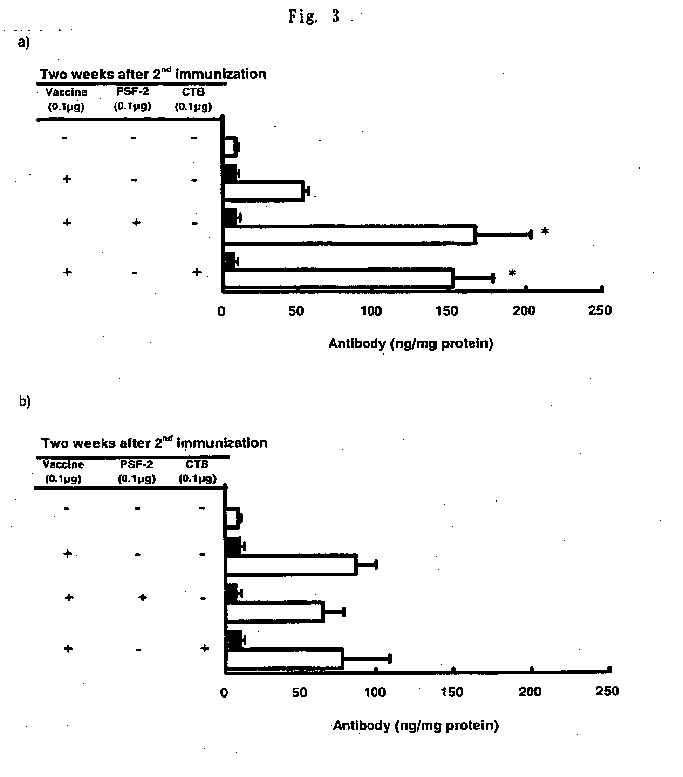

As shown in Figs. 3 (a) and (b), promoting effects of PSF-2 or CTB on production of anti-influenza specific antibodies were remarkable, similarly to the case of nasal administration of the vaccine, and there was no big difference between them. This immunity reinforcement effects were specific for IgA, and not found for IgG. In case of the subcutaneous injection, increase of IgA in the washing solution of the lung, but immunity reinforcement effects of PSF-2 or CTB were not found similarly to the case of the washing solution of the nasal cavity. Though not shown in the figure in this experiment, even in case that PSF-2 or CTB was administered nasally or subcutaneously as independent, respectively in the first immunization and the second immunization, increase of virus-specific IgA or IgG in the washing solution of the lung was not found in either case, and thus the effects of PSF-2 or CTB were determined as reinforcement effects for the vaccine actions. Though now shown in the figure, in the case of using PSF-1 or PSF-3 instead of PSF-2, similar effects were obtained. Similar phenomena were also found for PSF-4 and -5, but the effects were attenuated.

### EXAMPLE 4

### Influence of PSF-2 or CTB on production of anti-influenza specific antibodies (IgA and IgG) in the blood by influenza vaccine of (a) nasal administration and (b) subcutaneous-injection

As the nasal vaccine, 0.1 µg of the split-type influenza vaccine was administered to both noses of BALB/c mouse by 1 µl, respectively, i.e., 2 µl in total as a PBS solution, alone or with 0.1 µg of PSF-2 as the "AD vehicle", or 0.1 µg of CTB as an adjuvant. As the subcutaneous vaccine, the vaccine in the same amount as that of the nasal vaccine, PSF-2 or CTB was administered as 50 µl PBS solution to the hypoderma of the neck of BALB/c mouse. After 4 weeks, second immunization was conducted in the same manner as in the first immunization. To the control group, same amount of PBS was administered, respectively. 2 weeks after the second immunization, the mouse was slaughtered, blood collection from the heart was conducted, and the serum was prepared from this, and quantitization of the expression amount of anti-influenza antibodies was conducted using this (n=15-20; average±SE).

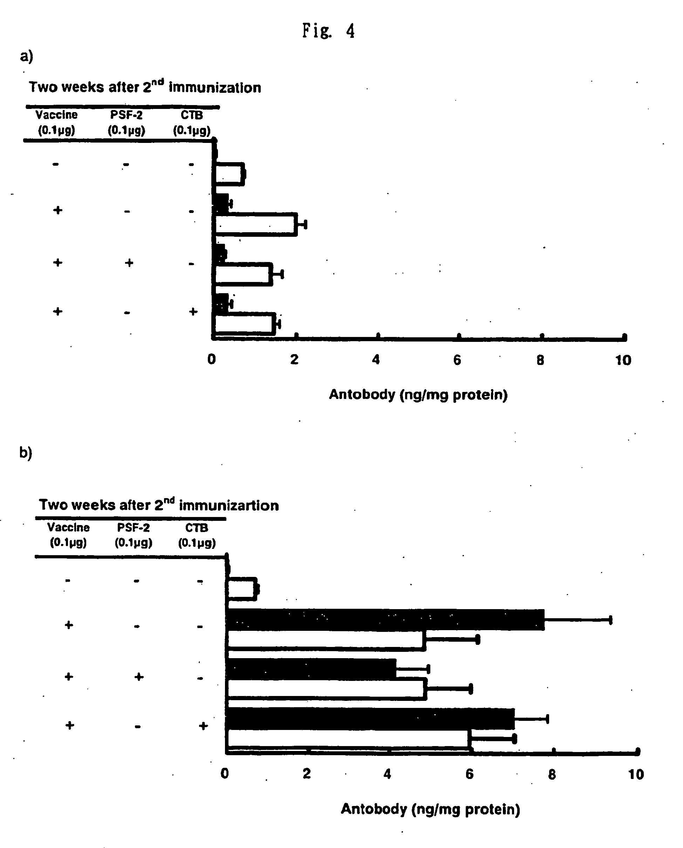

As shown in Figs. 4 (a) and (b), for the production amount of blood anti-influenza antibodies IgA (white bar) and IgG (black bar), slight increase of IgA or IgG was found in nasal vaccine administration, but increase of IgA or IgG by PSF-2 or CTB was not found, and thus immunity reinforcement effects were not found. On the other hand, in case of the subcutaneous injection, remarkable increase of IgG and definite increase of IgA were found by the split-type influenza vaccine, but also in this case, increase of antibody production by PSF-2 or CTB was not found and thus immunity reinforcement effects were not found. Particularly, in case of the subcutaneous injection, IgG increased specifically in blood. In the case of using PSF-1 instead of PSF-2, nearly similar effects were obtained (not shown in the figure).

In addition, even in case that PSF-2 or CTB was administered nasally or subcutaneously as independent, respectively in the first immunization and the second immunization, increase of virus-specific IgA or IgG in blood was not found, and thus the effects of PSF-2 or CTB were determined as reinforcement effects for the vaccine actions (not shown in the figure).

### EXAMPLE 5

### Influence of PSF-2 or CTB on antigen-presenting ability of dendritic cells of the nose, lung or spleen by influenza vaccine of (a) nasal administration and (b) subcutaneous-injection

As the nasal vaccine, 0.1 µg of the split-type influenza vaccine was administered to both noses of BALB/c mouse in 2 µl in total as 1 µl PBS solution, alone or with 0.1 µg of PSF-2, or 0.1 µg of CTB. As the subcutaneous vaccine, the split-type influenza vaccine in the same amount as that of the nasal vaccine, PSF-2 or CTB was administered as 50 µl PBS solution to the hypoderma of the neck of BALB/c mouse. After 2 days, the mouse was slaughtered, and the dendritic cells were prepared from the nose, lung or spleen, and expression level of MHC class II. CD40, B7-1 (CD80) and B7-2 (CD80) on the cell surface was measured by flow cytometry.

As a result, increase of expression of antigen-presenting related molecules. CD40 and B7-2 was found on the membrane surface of the dendritic cells (antigen-presenting cells) in the nose, where the vaccine had been challenged by CTB, and adjuvants effects were found in molecular level. In case of PSF-2, increase of expression of MHC II molecule was also found in addition to CD40 and B7-2 of the dendritic cells in the nose, and it was shown that at least three molecules of the dendritic cells are involved in the immunity reinforcement effects.

However, definite change was not found in CD40, B7-2 or MHC II molecule of the dendritic cells in the lung and spleen. In the case of using PSF-1 or PSF-3 instead of PSF-2, nearly similar effects were obtained. Similar phenomena were also found for PSF-4 and -5, but the effects were attenuated.

### EXAMPLE 6

### Influence of SPF-2 or CTB on TGF-β1 secretion level in the mucosal membrane of (a) the nasal cavity and (b) the alveoli by nasal administration of influenza vaccine

As the nasal vaccine, 0.1 µg of the split-type influenza vaccine was administered to both noses of BALB/c mouse in 2 µl in total as 1 µl PBS solution, alone or with 0.1 µg of SPF-2. or 0.1 µg of CTB. As the subcutaneous vaccine, the vaccine in the same amount as that of the nasal vaccine, PSF-2 or CTB was administered as 50 µl PBS solution to the hypoderma of the neck of BALB/c mouse. After 4 weeks, second immunization was conducted in the same manner as in the first immunization. To the control group, same amount of PBS was administered, respectively. 2 weeks after the second immunization, the mouse was slaughtered, and the washing solution of the nasal cavity was prepared, and using this, quantitization of TGF-β1 secretion amount was conducted (n=15-20; average±SE; *, the significance level by T-test was p<0.01 to the vaccine administration group).

It has been known that for differentiation of B cell to IgA-production cell (class switch), local TGF-β1 concentration present in the production cell is important (Stavnezer, J.: Regulation of antibody production and class switching by TGF-beta. J. Immunol. 155(4), 1647-1651, 1995). Herein, TGF-β1 concentration in a local mucosal membrane of (a) the nasal cavity or (b) the alveoli was investigated.

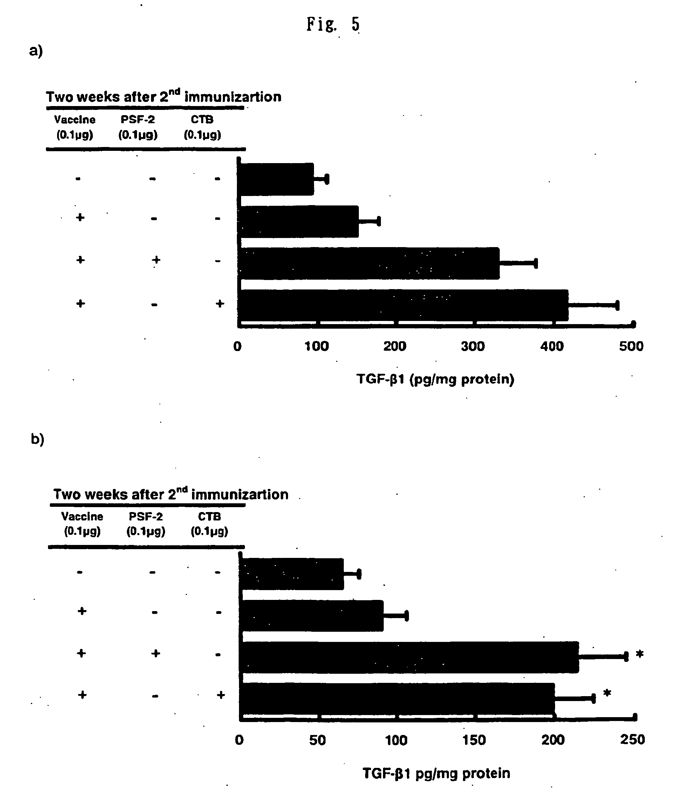

The TGF-β1 concentration in the local mucosal membrane of the nasal cavity or the alveoli where the split-type influenza vaccine was administered, increased significantly in presence of PSF-2 or CTB. The extent of increase was such that there was no significant difference between PSF-2 and CTB. The results are shown in Figs. 5 (a) and (b). It was found that PSF-2, which was derived from the living body, increased TGF-β1 concentration which urges promotion of differentiation of B cell secreting IgA as much as CTB, which is exogenous toxin. Though not shown in the figure, in the case of using PSF-1 or PSF-3 instead of PSF-2, nearly similar effects were obtained. Similar phenomena were also found for PSF-4 and -5, but the effects were attenuated. In addition, even in case that PSF-2 or CTB was administered nasally or subcutaneously as independent, respectively in the first immunization and the second immunization, increase of TGF-β1 concentration was not found, and thus the effects of PSF-2 or CTB were determined as reinforcement effects for the vaccine actions (not shown in the figure).

### EXAMPLE 7

### Influence of PSF-2 or CTB on production of anti-influenza specific antibodies (IgA and IgG) in (a) the nasal cavity, (b) the alveoli and (c) the blood by nasal administration of influenza vaccine

As the nasal vaccine, 0.2 µg of the split-type influenza vaccine was administered to both noses of BALB/c mouse by 1 µl, respectively. i.e., 2 µl in total as a PBS solution, alone or with 0.2 µg of PSF-2 as the "AD vehicle", or 0.2 µg of CTB as an adjuvant. After 4 weeks, second immunization was conducted in the same manner as in the first immunization. To the control group, same amount of PBS was administered, respectively. 2 weeks after the second immunization, the mouse was slaughtered in each group, and the washing solutions of the nasal cavity and the alveoli, and serum by blood collection from the heart were prepared, and using these, quantitization of expression amount of anti-influenza antibody was conducted (n=15-30; average±SE; +, p<0.01 vs. the vaccine independent administration).

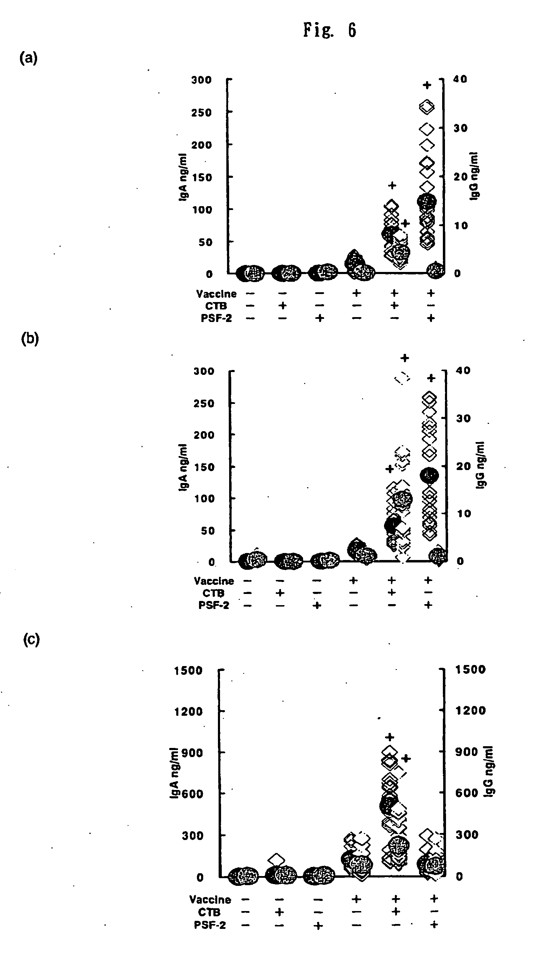

As shown in Figs. 6 (a) and (b), in the washing solutions of the nasal cavity and the alveoli, blood anti-influenza antibody IgA (blue circlet) showed remarkable increase in case that PSF-2 and the vaccine were administered. However, as shown in Fig. 6 (c), increase of IgG (red circlet) in blood was not found.

On the other hand, in case that CTB and the vaccine were administered, IgA and IgG increased in the washing solutions of the nasal cavity and the alveoli (Figs. 6 (a) and (b)), and also remarkable in blood (Fig. 6 (c)).

As described above, in case of CTB, it reacted with the antigen inoculated nasally, and caused systemic immune response, as well as establishment of local immunity, as reported conventionally. On the other hand, in case of PSF-2, it only established local mucosal immunity.

In addition, J. Freek van Iwaarden, et al. (Non-Patent Document 4) have reported that if macrophage is removed artificially from the lung, SP-B and lipid can induce systemic immune reaction, but in the case that macrophage is not removed, they cannot induce immunity. In addition, in this document, the amount of SP-B plus lipid necessary for induction of the systemic immune reaction, is 250 to 300 µl, which is quite different from the PSF-2 administration amount in the above-mentioned Examples (0.2 µl). However, it has never mentioned local mucosal immunity.

### EXAMPLE 8

### Influence of SPF-2 or CTB on various cytokines secreted from the nose, lung or lymphocyte of spleen by nasal administration of influenza vaccine

As the nasal vaccine, 0.2 µg of the split-type influenza vaccine was administered to the upper respiratory tract of BALB/c mouse in 2 µl in total as 1 µl PBS solution, alone or with 0.2 µg of PSF-2 or 0.2 µg of CTB. 2 weeks after the second immunization, the mouse was slaughtered, and quantitization of secretion amount of TGF-β1 and cytokines (IL-4, IL-5,IL-6 and IL-13) from the nose, lung or lymphocyte of spleen was conducted (n=15-20: average±SE; +++, p=0.06; ++, P=0.05; +, P=0.01 vs. the vaccine independent administration).

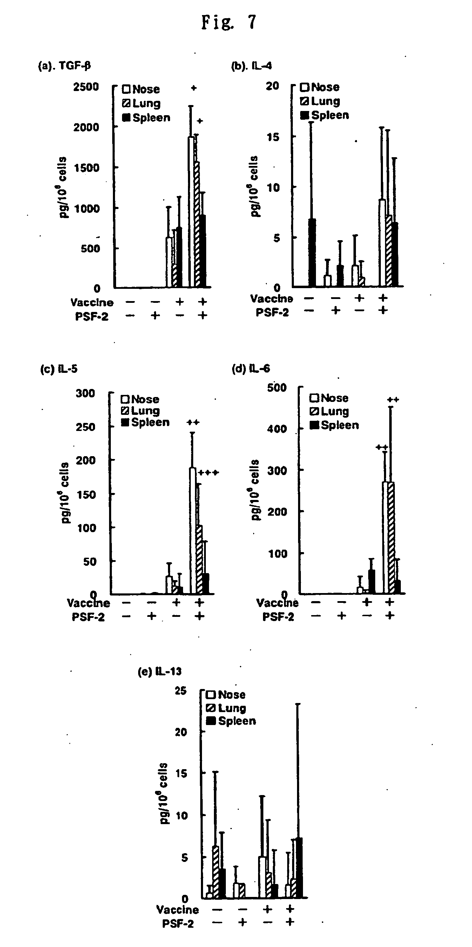

As shown in Figs. 7 (a) to (e), TGF-β1, IL-5 and IL-6 were found to increase significantly in secretion, whereas IL-4 and IL-13 were found to increase significantly in the local mucosal membrane (nose, lung) after the vaccine immunity along with PSF-2. On the other hand, significant increase of any cytokine was not observed in the spleen.

From the above results, it was found that PSF-2 increase Th2-type cytokine, which promotes differentiation and induction of B cell producing IgA.

### EXAMPLE 9

### Influence of PSF-3 on production of anti-influenza specific antibodies (IgA and IgG) in (a) the nasal cavity, (b) the alveoli and (c) the blood by nasal administration of influenza vaccine

As the nasal vaccine, 0.2 µg of the split-type influenza vaccine was administered to both noses of BALB/c mouse by 1 µl, respectively, i.e., 2 µl in total as a PBS solution, alone or with 0.2 µg of PSF-3 (a mixture of same amount of SP-B fragment, i.e., 253 to 278 peptides described in SEQ ID No. 16, and SP-C fragment, i.e., 24 to 58 peptides described in SEQ ID No. 21) as the "AD vehicle", or 0.2 µg of lipid ingredient. After 4 weeks, second immunization was conducted in the same manner as in the first immunization. To the control group, same amount of PBS was administered, respectively. 2 weeks after the second immunization, the mouse was slaughtered in each group, and the washing solutions of the nasal cavity and the alveoli, and serum by blood collection from the heart were prepared, and using these, quantization of expression amount of anti-influenza antibody was conducted (n=15-30: average±SE; +, p<0.01 vs. the vaccine independent administration).

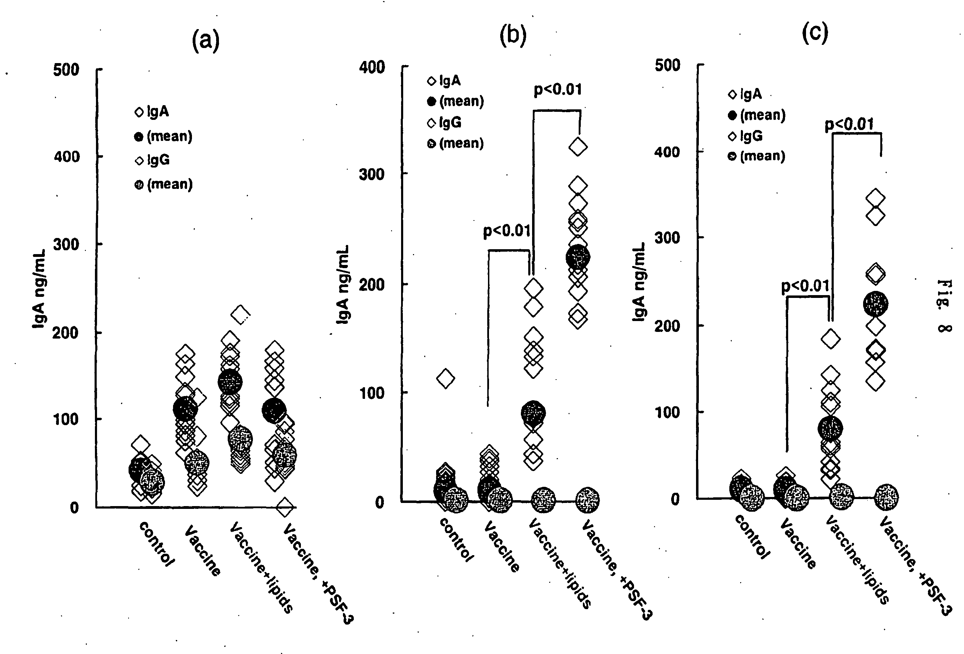

As shown in Figs. 8 (a) and (b), in the washing solutions of the nasal cavity and the alveoli, blood anti-influenza antibody IgA (blue circlet) showed remarkable increase in case that PSF-3 and the vaccine were administered, but significant increase of IgG (red circlet) was not found. On the other hand, in serum (c), neither IgA (blue circlet) nor IgG (red circlet) was found to increase significantly.

### Industrial Applicability

The "AD vehicle" disclosed herein, exerts a function of transporting all the substances such as antigen, drug, nutrient, and the like from the mucosal membrane of the nose, trachea, intestine, and the like, or the skin into cells, and also induces preferential and selective production of IgA, enabling a mucosal vaccine, prevention and treatment of allergy, transmucosal and transdermal DDS, and transmucosal and transdermal administration of useful substance such as a drug, nutrient, and the like. Furthermore, it has been approved already for its clinical use and safety in this country or other countries.

Accordingly, application and use of the "AD vehicle" is expected in very broad range of industries such as medication/pharmaceutics in biological preparations, DDS, and the like, food and drink industries in functional food, health food, and the like, agriculture and agricultural chemicals in raising and cultivation of agricultural products, anti-disease measures, insect destruction, and the like, cultivation fishery in fish-disease vaccine and administration thereof, and the like, architecture or environment preservation in anti-ant, anti-insect, and the like.

## Claims

1. A mucosal vaccine comprising:
(i) an antigen-drug vehicle, which is a complex comprising:
a pulmonary surfactant protein B or a fragment of protein B that is a peptide comprising the amino acid sequence of SEQ ID No. 2, 7, 8, 10, 11, 12, 13, 14, 15, 16, 17, 18, 19 or 20, a pulmonary surfactant protein C or a fragment of protein C that is a peptide comprising the amino acid sequence of SEQ ID No. 4, 9, 21 or 6, and at least one lipid; and
(ii) an antigen;
wherein said mucosal vaccine induces mucosal immunity.

2. The mucosal vaccine according to claim 1, wherein the at least one lipid is selected from a lipid group consisting of phosphatidyl choline, dipalmitoyl phosphatidyl choline, phosphatidyl serine, dipalmitoyl glycerophosphocholine, diacyl glycerophosphoglycerol, phosphatidylglycerol, phosphatidyl inositol, phosphatidyl ethanolamine, phosphatidic acid, lauric acid, myristic acid, palmitic acid, stearic acid and oleic acid.

3. The mucosal-vaccine according to claim 1 or claim 2, wherein the lipid is a membrane of a sheet form or a roll form, and multiple chains of at least one fragment selected from pulmonary surfactant protein B or multiple fragments of protein B as defined in claim 1, and at least one fragment selected from pulmonary surfactant protein C or multiple fragments of protein C as defined in claim 1, are implanted in a spike shape with the ends of the hydrophobic area being intrusive into the lipid membrane.

4. The mucosal vaccine according to any one of claims 1 to 3, wherein the induction of mucosal immunity is made by promoting the production of an IgA antibody in a local mucosal membrane.

5. The mucosal vaccine according to any one of claims 1 to 4, wherein the induction of mucosal immunity is made by promoting the production of TGF-β1 and Th2 type cytokine in a local mucosal membrane.

6. An agent suitable for prevention or treatment of allergy, comprising an antigen-drug vehicle as defined in any one of claims 1 to 3 and an allergen, wherein said agent induces mucosal immunity.

7. The agent suitable for prevention or treatment of allergy according to claim 6, wherein the induction of mucosal immunity is made by promoting the production of an IgA antibody in a local mucosal membrane.

8. The agent suitable for prevention or treatment of allergy according to claim 6 or 7, wherein the induction of mucosal immunity is made by promoting the production of TGF-β1 and Th2 type cytokine in a local mucosal membrane.

9. The mucosal vaccine or agent, as defined in any of claims 1 to 8 for use in therapy.

10. The mucosal vaccine or agent of claim 9 for use in inducing mucosal immunity or for prevention or treatment of allergy.

11. The use of a mucosal vaccine according to any one of claims 1 to 3, in the preparation of a medicament for inducing mucosal immunity, wherein said mucosal vaccine is for administration into the nasal cavity or upper respiratory tract.

12. The use of an agent according to claim 6, in the preparation of a medicament for prevention or treatment of allergy, wherein said agent induces mucosal immunity.

13. The mucosal vaccine, agent or use according to any one of claims 10 to 12, wherein the induction of mucosal immunity is made by promoting the production of an IgA antibody in a local mucosal membrane.

14. The mucosal vaccine, agent or use according to any one of claims 10 to 12, wherein the induction of mucosal immunity is made by promoting the production of TGF-β1 and Th2 type cytokine in a local mucosal membrane.

## Patentansprüche

1. Schleimhautimpfstoff, der enthält:
(i) einen Antigen-Arzneimittelträger, der ein Komplex ist, der enthält:
ein oberflächenaktives Lungenprotein B oder ein Fragment des Proteins B, das ein Peptid mit der Aminosäuresequenz von SEQ ID NR. 2, 7, 8, 10, 11, 12, 13, 14, 15, 16, 17, 18, 19 oder 20 ist, ein oberflächenaktives Lungenprotein C oder ein Fragment des Proteins C, das ein Peptid mit der Aminosäuresequenz von SEQ ID NR. 4, 9, 21 oder 6 ist, und mindestens ein Lipid; und
(ii) ein Antigen;
wobei der Schleimhautimpfstoff Schleimhautimmunität hervorruft.

2. Schleimhautimpfstoff nach Anspruch 1, wobei das mindestens eine Lipid aus einer Lipidgruppe ausgewählt ist, die aus Phosphatidylcholin, Dipalmitoylphosphatidylcholin, Phosphatidylserin, Dipalmitoylglycerophosphocholin, Diacylglycerophosphoglycerin, Phosphatidylglycerin, Phosphatidylinositol, Phosphatidylethanolamin, Phosphatidsäure, Laurinsäure, Myristinsäure, Palmitinsäure, Stearinsäure und Ölsäure besteht.

3. Schleimhautimpfstoff nach Anspruch 1 oder Anspruch 2, wobei das Lipid eine Membran in einer Schichtform oder einer Rollenform ist und mehrere Ketten von mindestens einem Fragment, das aus dem oberflächenaktiven Lungenprotein B oder mehreren Fragmenten des Proteins B, wie in Anspruch 1 definiert, ausgewählt ist, und von mindestens einem Fragment, das aus dem oberflächenaktiven Lungenprotein C oder mehreren Fragmenten des Proteins C, wie in Anspruch 1 definiert, ausgewählt ist, in einer Nadelform implantiert werden, wobei die Enden des hydrophoben Bereichs in die Lipidmembran eindringen.

4. Schleimhautimpfstoff nach einem der Ansprüche 1 bis 3, wobei das Hervorrufen von Schleimhautimmunität durch Fördern der Erzeugung eines IgA-Antikörpers in einer lokalen Schleimhautmembran durchgeführt wird.

5. Schleimhautimpfstoff nach einem der Ansprüche 1 bis 4, wobei das Hervorrufen von Schleimhautimmunität durch Fördern der Erzeugung von Zytokin vom Typ TGF-β1 und Th2 in einer lokalen Schleimhautmembran durchgeführt wird.

6. Mittel, das zur Verhinderung oder Behandlung einer Allergie geeignet ist, mit einem Antigen-Arzneimittelträger, wie in einem der Ansprüche 1 bis 3 definiert, und einem Allergen, wobei das Mittel Schleimhautimmunität hervorruft.

7. Mittel, das zur Verhinderung oder Behandlung einer Allergie geeignet ist, nach Anspruch 6, wobei das Hervorrufen von Schleimhautimmunität durch Fördern der Erzeugung eines IgA-Antikörpers in einer lokalen Schleimhautmembran durchgeführt wird.

8. Mittel, das zur Verhinderung oder Behandlung einer Allergie geeignet ist, nach Anspruch 6 oder 7, wobei das Hervorrufen von Schleimhautimmunität durch Fördern der Erzeugung von Zytokin vom Typ TGF-β1 und Th2 in einer lokalen Schleimhautmembran durchgeführt wird.

9. Schleimhautimpfstoff oder Mittel nach einem der Ansprüche 1 bis 8 zur Verwendung bei einer Therapie.

10. Schleimhautimpfstoff oder Mittel nach Anspruch 9 zur Verwendung beim Hervorrufen von Schleimhautimmunität oder für die Verhinderung oder Behandlung einer Allergie.

11. Verwendung eines Schleimhautimpfstoffs nach einem der Ansprüche 1 bis 3 bei der Herstellung eines Medikaments zum Hervorrufen von Schleimhautimmunität, wobei der Schleimhautimpfstoff zur Verabreichung in die Nasenhöhle oder an die oberen Atemwege dient.

12. Verwendung eines Mittels nach Anspruch 6 bei der Herstellung eines Medikaments für die Verhinderung oder Behandlung einer Allergie, wobei das Mittel Schleimhautimmunität hervorruft.

13. Schleimhautimpfstoff, Mittel oder Verwendung nach einem der Ansprüche 10 bis 12, wobei das Hervorrufen von Schleimhautimmunität durch Fördern der Erzeugung eines IgA-Antikörpers in einer lokalen Schleimhautmembran durchgeführt wird.

14. Schleimhautimpfstoff, Mittel oder Verwendung nach einem der Ansprüche 10 bis 12, wobei das Hervorrufen von Schleimhautimmunität durch Fördern der Erzeugung von Zytokin vom Typ TGF-β1 und Th2 in einer lokalen Schleimhautmembran durchgeführt wird.

## Revendications

1. Vaccin muqueux comportant :
(i) un véhicule de médicament-antigène, lequel véhicule est un complexe comportant :
une protéine B du surfactant pulmonaire ou un fragment de protéine B qui est un peptide comportant la séquence d'acides aminés des SEQ ID N° 2, 7, 8, 10, 11, 12, 13, 14, 15, 16, 17, 18, 19 ou 20, une protéine C du surfactant pulmonaire ou un fragment de protéine C qui est un peptide comportant la séquence d'acides aminés des SEQ ID N° 4, 9, 21 ou 6, et au moins un lipide ; et
(ii) un antigène ;
dans lequel ledit vaccin muqueux induit une immunité muqueuse.

2. Vaccin muqueux selon la revendication 1, dans lequel le au moins un lipide est choisi parmi un groupe de lipides constitué de phosphatidyl choline, dipalmitoyl phosphatidyl choline, phosphatidyl sérine, dipalmitoyl glycérophosphocholine, diacyl glycérophosphoglycérol, phosphatidylglycérol, phosphatidyl inositol, phosphatidyl éthanolamine, acide phosphatidique, acide laurique, acide myristique, acide palmitique, acide stéarique et acide oléique.

3. Vaccin muqueux selon la revendication 1 ou la revendication 2, dans lequel le lipide est une membrane ayant une forme de feuille ou une forme de rouleau, et de multiples chaînes d'au moins un fragment choisi parmi la protéine B du surfactant pulmonaire ou de multiples fragments de protéine B tel que défini dans la revendication 1, et au moins un fragment choisi parmi la protéine C du surfactant pulmonaire ou de multiples fragments de protéine C tel que défini dans la revendication 1, sont implantés en forme de pointe, les extrémités de la zone hydrophobe étant intrusives dans la membrane lipidique.

4. Vaccin muqueux selon l'une quelconque des revendications 1 à 3, dans lequel l'induction d'une immunité muqueuse est réalisée en favorisant la production d'un anticorps IgA dans une membrane muqueuse locale.

5. Vaccin muqueux selon l'une quelconque des revendications 1 à 4, dans lequel l'induction d'une immunité muqueuse est réalisée en favorisant la production de cytokine de type TGF-β1 et Th2 dans une membrane muqueuse locale.

6. Agent adapté pour la prévention ou le traitement d'une allergie, comportant un véhicule de médicament-antigène tel que défini dans l'une quelconque des revendications 1 à 3 et un allergène, dans lequel ledit agent induit une immunité muqueuse.

7. Agent adapté pour la prévention ou le traitement d'une allergie selon la revendication 6, dans lequel l'induction d'une immunité muqueuse est réalisée en favorisant la production d'un anticorps IgA dans une membrane muqueuse locale.

8. Agent adapté pour la prévention ou le traitement d'une allergie selon la revendication 6 ou 7, dans lequel l'induction d'une immunité muqueuse est réalisée en favorisant la production de cytokine de type TGF-β1 et Th2 dans une membrane muqueuse locale.

9. Vaccin muqueux ou agent, tel que défini dans l'une quelconque des revendications 1 à 8 pour une utilisation en thérapie.

10. Vaccin muqueux ou agent selon la revendication 9 pour une utilisation dans l'induction d'une immunité muqueuse ou pour la prévention ou le traitement d'une allergie.

11. Utilisation d'un vaccin muqueux selon l'une quelconque des revendications 1 à 3, dans la préparation d'un médicament destiné à induire une immunité muqueuse, dans lequel ledit vaccin muqueux est destiné à une administration dans la cavité nasale ou dans les voies respiratoires supérieures.

12. Utilisation d'un agent selon la revendication 6, dans la préparation d'un médicament destiné à la prévention ou au traitement d'une allergie, dans lequel ledit agent induit une immunité muqueuse.

13. Vaccin muqueux, agent ou utilisation selon l'une quelconque des revendications 10 à 12, dans lequel l'induction d'une immunité muqueuse est réalisée en favorisant la production d'un anticorps IgA dans une membrane muqueuse locale.

14. Vaccin muqueux, agent ou utilisation selon l'une quelconque des revendications 10 à 12, dans lequel l'induction d'une immunité muqueuse est réalisée en favorisant la production de cytokine de type TGF-β1 et Th2 dans une membrane muqueuse locale.


### SEQUENCE LISTING

<110> The University of Tokushima
<120> Antigen-Drug Vehicle Enabling Transmucosal and Transdermal Administration and Method of Inducing Mucosal Immunity, Mucosal Vaccine and DDS using The Same.
<130> 69.60.88052
<140> EPA 05721586.5
   <141> 2005-03-22
<150> JP 2004-111249
   <151> 2004-04-05
<150> JP 2004-125036
   <151> 2004-04-21
<160> 21
<170> Patent ln version 3.1
<210> 1
   <211> 1146
   <212> DNA
   <213> Homo sapiens
<220>
   <221> CDS
   <222> (1)...(1143)
   <223>
<400> 1
atg gct gag tca cac ctg ctg cag tgg ctg ctg ctg ctg ctg ccc acg 48
   ctc tgt ggc cca ggc act gct gcc tgg acc acc tca tcc ttg gcc tgt 96
   gcc cag ggc cct gag ttc tgg tgc caa agc ctg gag caa gca ttg cag 144
   tgc aga gcc cta ggg cat tgc cta cag gaa gtc tgg gga cat gtg gga 192
   gcc gat gac cta tgc caa gag tgt gag gac atc gtc cac atc ctt aac 240
   aag atg gcc aag gag gcc att ttc cag gac acg atg agg aag ttc ctg 288
   gag cag gag tgc aac gtc ctc ccc ttg aag ctg ctc atg ccc cag tgc 336
   aac caa gtg ctt gac gac tac ttc ccc ctg gtc atc gac tac ttc cag 384
   aac cag act gac tca aac ggc atc tgt atg cac ctg ggc ctg tgc aaa 432
   tcc cgg cag cca gag cca gag cag gag cca ggg atg tca gac ccc ctg 480
   ccc aaa cct ctg cgg gac cct ctg cca gac cct ctg ctg gac aag ctc 528
   gtc ctc cct gtg ctg ccc ggg gcc ctc cag gcg agg cct ggg cct cac 576
   aca cag gat ctc tcc gag cag caa ttc ccc att cct ctc ccc tat tgc 624
   tgg ctc tgc agg gct ctg atc aag cgg atc caa gcc atg att ccc aag 672
   ggt gcg cta gct gtg gca gtg gcc cag gtg tgc cgc gtg gta cct ctg      720
   gtg gcg ggc ggc atc tgc cag tgc ctg gct gag cgc tac tcc gtc atc      768
   ctg ctc gac acg ctg ctg ggc cgc atg ctg ccc cag ctg gtc tgc cgc      816
   ctc gtc ctc cgg tgc tcc atg gat gac agc gct ggc cca agg tcg ccg      864
   aca gga gaa tgg ctg ccg cga gac tct gag tgc cac ctc tgc atg tcc      912
   gtg acc acc cag gcc ggg aac agc agc gag cag gcc ata cca cag gca      960
   atg ctc cag gcc tgt gtt ggc tcc tgg ctg gac agg gaa aag tgc aag     1008
   caa ttt gtg gag cag cac acg ccc cag ctg ctg acc ctg gtg ccc agg     1056
   ggc tgg gat gcc cac acc acc tgc cag gcc ctc ggg gtg tgt ggg acc     1104
   atg tcc agc cct ctc cag tgt atc cac agc ccc gac ctt tga             1146
<210> 2
   <211> 381
   <212> PRT
   <213> Homo sapiens
<400> 2
<210> 3
   <211> 594
   <212> DNA
   <213> Homo sapiens
<220>
   <221> CDS
   <222> (1).. (591)
   <223>
<400> 3
   atg gat gtg ggc agc aaa gag gtc ctg atg gag agc ccg ccg gac tac 48
   tcc gca gct ccc cgg ggc cga ttt ggc att ccc tgc tgc cca gtg cac 96
   ctg aaa cgc ctt ctt atc gtg gtg gtg gtg gtg gtc ctc atc gtc gtg 144
   gtg att gtg gga gcc ctg ctc atg ggt ctc cac atg agc cag aaa cac 192
   acg gag atg gtt ctg gag atg agc att ggg gcg ccg gaa gcc cag caa 240
   cgc ctg gcc ctg agt gag cac ctg gtt acc act gcc acc ttc tcc atc 288
   ggc tcc act ggc ctc gtg gtg tat gac tac cag cag ctg ctg atc gcc 336
   tac aag cca gcc cct ggc acc tgc tgc tac atc atg aag ata gct cca 384
   gag agc atc ccc agt ctt gag gct ctc act aga aaa gtc cac aac ttc 432
   cag atg gaa tgc tct ctg cag gcc aag ccc gca gtg cct acg tct aag 480
   ctg ggc cag gca gag ggg cga gat gca ggc tca gca ccc tcc gga ggg 528
   gac ccg gcc ttc ctg ggc atg gcc gtg aac acc ctg tgt ggc gag gtg 576
   ccg ctc tac tac atc tag 594
<210> 4
   <211> 197
   <212> PRT
   <213> Homo sapiens
<400> 4
<210> 5
   <211> 576
   <212> DNA
   <213> Homo sapiens
<220>
   <221> CDS
   <222> (1).. (573)
   <223>
<400> 5
   atg gat gtg ggc agc aaa gag gtc ctg atg gag agc ccg ccg gac tac 48
   tcc gca gct ccc cgg ggc cga ttt ggc att ccc tgc tgc cca gtg cac 96
   ctg aaa cgc ctt ctt atc gtg gtg gtg gtg gtg gtc ctc atc gtc gtg 144
   gtg att gtg gga gcc ctg ctc atg ggt ctc cac atg agc cag aaa cac 192
   acg gag atg gtt ctg gag atg agc att ggg gcg ccg gaa gcc cag caa 240
   cgc ctg gcc ctg agt gag cac ctg gtt acc act gcc acc ttc tcc atc 288
   ggc tcc act ggc ctc gtg gtg tat gac tac cag cag ctg ctg atc gcc 336
   tac aag cca gcc cct ggc acc tgc tgc tac atc atg aag ata gct cca 384
   gag agc atc ccc agt ctt gag gct ctc act aga aaa gtc cac aac ttc 432
   cag gcc aag ccc gca gtg cct acg tct aag ctg ggc cag gca gag ggg 480
   cga gat gca ggc tca gca ccc tcc gga ggg gac ccg gcc ttc ctg ggc 528
   atg gcc gtg aac acc ctg tgt ggc gag gtg ccg ctc tac tac atc tag 576
<210> 6
   <211> 191
   <212> PRT
   <213> Homo sapiens
<400> 6
<210> 7
   <211> 12
   <212> PRT
   <213> Homo sapiens
<400> 7
   Leu Ile Lys Arg Ile Gln Ala Met Ile Pro Lys Gly
   1 5 10
<210> 8
   <211> 10
   <212> PRT
   <213> Homo sapiens
<400> 8
   Leu Leu Asp Thr Leu Leu Gly Arg Met Leu
   1 5 10
<210> 9
   <211> 30
   <212> PRT
   <213> Homo sapiens
<400> 9
   Cys Pro Val His Leu Lys Arg Leu Leu Ile Val Val Val Val Val Val
   1 5 10 15
   Leu Ile Val Val Val Ile Val Gly Ala Leu Leu Met Gly Leu
   20 25 30
<210> 10
   <211> 20
   <212> PRT
   <213> Homo sapiens
<400> 10
   Met Ala Glu Ser His Leu Leu Gln Trp Leu Leu Leu Leu Leu Pro Thr
   1 5 10 15
   Leu Cys Gly Pro
   20
<210> 11
   <211> 9
   <212> PRT
   <213> Homo sapiens
<400> 11
   Val Leu Pro Leu Lys Leu Leu Met Pro
   1 5
<210> 12
   <211> 9
   <212> PRT
   <213> Homo sapiens
<400> 12
   Tyr Phe Pro Leu Val Ile Asp Tyr Phe
   1 5
<210> 13
   <211> 7
   <212> PRT
   <213> Homo sapiens
<400> 13
   Ile Cys Met His Leu Gly Leu
   1 5
<210> 14
   <211> 15
   <212> PRT
   <213> Homo sapiens
<400> 14
   Pro Leu Leu Asp Lys Leu Val Leu Pro Val Leu Pro Gly Ala Leu
   1 5 10 15
<210> 15
   <211> 79
   <212> PRT
   <213> Homo sapiens
<400> 15
   Phe Pro Ile Pro Leu Pro Tyr Cys Trp Leu Cys Arg Ala Leu Ile Lys
   1 5 10 15
   Arg Ile Gln Ala Met Ile Pro Lys Gly Ala Leu Ala Val Ala Val Ala
   20 25 30
   Gln Val Cys Arg Val Val Pro Leu Val Ala Gly Gly Ile Cys Gln Cys
   35 40 45
   Leu Ala Glu Arg Tyr Ser Val Ile Leu Leu Asp Thr Leu Leu Gly Arg
   50 55 60
   Met Leu Pro Gln Leu Val Cys Arg Leu Val Leu Arg Cys Ser Met
   65 70 75
<210> 16
   <211> 26
   <212> PRT
   <213> Homo sapiens
<400> 16
   Tyr Ser Val Ile Leu Leu Asp Thr Leu Leu Gly Arg Met Leu Pro Gln
   1 5 10 15
   Leu Val Cys Arg Leu Val Leu Arg Cys Ser
   20 25
<210> 17
   <211> 8
   <212> PRT
   <213> Homo sapiens
<400> 17
   His Leu Cys Met Ser Val Thr Thr
   1 5
<210> 18
   <211> 14
   <212> PRT
   <213> Homo sapiens
<400> 18
   Ile Pro Gln Ala Met Leu Gln Ala Cys Val Gly Ser Trp Leu
   1 5 10
<210> 19
   <211> 8
   <212> PRT
   <213> Homo sapiens
<400> 19
   Pro Gln Leu Leu Thr Leu Val Pro
   1 5
<210> 20
   <211> 24
   <212> PRT
   <213> Homo sapiens
<400> 20
   Thr Thr Cys Gln Ala Leu Gly Val Cys Gly Thr Met Ser Ser Pro Leu
   1 5 10 15
   Gln Cys Ile His Ser Pro Asp Leu
   20
<210> 21
   <211> 35
   <212> PRT
   <213> Homo sapiens
<400> 21
   Phe Gly Ile Pro Cys Cys Pro Val His Leu Lys Arg Leu Leu Ile Val
   1 5 10 15
   Val Val Val Val Val Leu Ile Val Val Val Ile Val Gly Ala Leu Leu
   20 25 30
   Met Gly Leu
   35